(19) Europäisches Patentamt
European Patent Office
Office européen des brevets

(11) Veröffentlichungsnummer : **0 018 497**
**B1**

(12) # EUROPÄISCHE PATENTSCHRIFT

(45) Veröffentlichungstag der Patentschrift : **28.04.82**

(21) Anmeldenummer : **80101586.8**

(22) Anmeldetag : **26.03.80**

(51) Int. Cl.³ : **C 07 D277/34**, C 07 D261/12, C 07 D263/38, C 07 D271/06, C 07 D271/10, C 07 D275/02, C 07 D285/08, C 07 D285/10, C 07 D285/12, C 07 D413/12, C 07 D417/12 // (C07D487/08, 223/00, 209/00),(C07D491/10, 317/00, 221/00)

(54) **Azolyloxy-essigsäureamide, Verfahren zu ihrer Herstellung und ihre Verwendung als Herbizide.**

(30) Priorität : **06.04.79 DE 2914003**
**06.02.80 DE 3004326**

(43) Veröffentlichungstag der Anmeldung : **12.11.80 (Patentblatt 80/23)**

(45) Bekanntmachung des Hinweises auf die Patenterteilung : **28.04.82 Patentblatt 82/17**

(84) Benannte Vertragsstaaten : **AT BE CH DE FR GB IT NL SE**

(56) Entgegenhaltungen :
**EP - A2 - 0 005 501**
**FR - A - 1 313 840**
**US - A - 4 067 725**

(73) Patentinhaber : **BAYER AG**
**Zentralbereich Patente, Marken und Lizenzen**
**D-5090 Leverkusen 1, Bayerwerk (DE)**

(72) Erfinder : **Förster, Heinz, Dr.**
**Am Eckbusch 47**
**D-5600 Wuppertal 1 (DE)**
Erfinder : **Hofer, Wolfgang, Dr.**
**Pahlkestrasse 59**
**D-5600 Wuppertal 1 (DE)**
Erfinder : **Mues, Volker, Dr.**
**Gellertweg 13**
**D-5600 Wuppertal 1 (DE)**
Erfinder : **Eue, Ludwig, Dr.**
**Paul-Klee-Strasse 36**
**D-5090 Leverkusen 1 (DE)**
Erfinder : **Schmidt, Robert Rudolf, Dr.**
**Hahnenweg 5**
**D-5000 Köln 80 (DE)**

**EP 0 018 497 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Azolyloxyessigsäureamide, Verfahren zu ihrer Herstellung und ihre Verwendung als Herbizide

Die Erfindung betrifft neue Azolyloxyessigsäureamide, ein Verfahren zu ihrer Herstellung sowie ihre Verwendung als Herbizide.

Es ist bereits bekannt geworden, daß bestimmte Phenoxycarbonsäureamide, wir z.B. 2,4-Dichlorphenoxy-essigsäureamid, herbizid wirksam sind (vgl. FR-A 1 313 840). Die als Herbizide bekannten Phenoxycarbonsäureamide zeigen jedoch bei den üblichen Aufwandmengen nur eine geringe Wirkung gegen Ungräser und können zur Bekämpfung von Unkräutern in verschiedenen dikotylen Kulturen wegen mangelnder Selektivität nicht verwendet werden.

Es wurden nun neue Azolyloxyessigsäureamide der Formel I

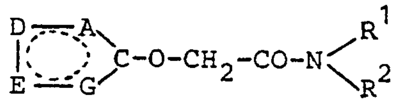

(I)

aufgefunden, in welcher

A für C-$R^3$ oder N steht,
D für C-$R^4$ oder N steht,
E für C-$R^5$, N, O oder S steht und
G für C-$R^6$, N, O oder S steht,

mit der Maßgabe, daß wenigstens eines der Ringglieder (A, D, E oder G) für N steht und mindestens eines der Ringglieder E oder G für O oder S steht;
in welcher weiter

$R^1$ und $R^2$, welche gleich oder verschieden sein können, einzeln für Wasserstoff, Alkyl, Cyanoalkyl, Alkoxyalkyl, Alkylthioalkyl, Alkenyl, Alkinyl, jeweils mit bis zu 10 Kohlenstoffatomen, Cycloalkyl mit bis zu 12 Kohlenstoffatomen, Aralkyl mit 1 oder 6 Kohlenstoffatomen im Alkylteil und 6 oder 10 Kohlenstoffatomen im Arylteil, welcher gegebenenfalls durch Halogen substituiert ist, oder für Aryl mit 6 oder 10 Kohlenstoffatomen stehen, wobei der Arylrest durch 1 bis 3 Halogenatome, 1 bis 3 Alkyl- oder Halogenalkyl-Gruppen mit jeweils 1 bis 4 Kohlenstoffatomen, Nitro, Cyano oder Alkoxy mit 1 bis 4 Kohlenstoffatomen substituiert sein kann,

oder worin

Die Reste $R^1$ und $R^2$ zusammen mit dem Stickstoffatom, as das sie gebunden sind, einen gegebenenfalls durch 1 bis 3 Alkylgruppen mit jeweils 1 bis 5 Kohlenstoffatomen oder durch zwei geminale Alkoxygruppen mit jeweils 1 bis 3 Kohlenstoffatomen substituierten oder gegebenenfalls durch Dioxolanyliden- oder Dioxanyliden-reste spiro-cyclischverknüpft substituierten, gegebenenfalls teilweise ungesättigten und/oder benzannellierten Monocyclus oder Bicyclus mit bis zu 15 Kohlenstoffatomen bilden, oder worin die Reste $R^1$ und $R^2$ zusammen mit dem Stickstoffatom, an das sie gebunden sind, einen gegebenenfalls durch 1 bis 3 Alkylgruppen mit jeweils 1 bis 5 Kohlenstoffatomen, durch Phenyl, welches gegebenenfalls durch $C_1$-$C_4$-Alkyl, $C_1$-$C_4$-Alkoxy, Halogen, $C_1$-$C_2$-Halogenalkyl oder Nitro substituiert ist, durch Benzyl oder Phenyläthyl substituierten, gesättigten und ein weiteres Stickstoffatom, Sauerstoffatom oder Schwefelatom enthaltenden Monocyclus mit bis zu 5 Kohlenstoffatomen bilden;
in welcher ferner $R^3$, $R^4$, $R^5$ und $R^6$, welche gleich oder verschieden sein können, einzeln für Wasserstoff, Halogen, Nitro, Cyano, Amino, $C_1$-$C_4$-Alkylamino, Di-$C_1$-$C_4$-alkylamino, $C_1$-$C_4$-Alkylcarbonylamino, $C_1$-$C_4$-Alkyl-carbonyl, Carboxy, $C_1$-$C_4$-Alkoxy-carbonyl, Carbamoyl, $C_1$-$C_4$-Alkylamino-carbonyl, Di-$C_1$-$C_4$-alkyl-amino-carbonyl, für gegebenenfalls durch Halogen, Nitro oder $C_1$-$C_4$-Alkyl substituiertes Phenyl-amino-carbonyl, für gegebenenfalls durch Halogen, Nitro, Cyano, $C_1$-$C_4$-Alkyl, $C_1$-$C_4$-Halogenalkyl oder $C_1$-$C_4$-Alkoxy substituiertes Phenyl, für gegebenenfalls durch Halogen substituiertes Benzyl oder Phenylethyl, für gegebenenfalls durch Halogen substituiertes $C_1$-$C_4$-Alkoxy, $C_2$-$C_4$-Alkenoxy, $C_2$-$C_4$-Alkinoxy, $C_1$-$C_4$-Alkoxy-carbonyl-methoxy, Benzyloxy oder Phenoxy, für gegebenenfalls halogen-substituiertes $C_1$-$C_4$-Alkylthio, $C_2$-$C_4$-Alkenylthio, $C_2$-$C_4$-Alkinylthio, $C_1$-$C_4$-Alkoxycarbonyl-methylthio, Benzylthio, Phenylthio, $C_1$-$C_4$-Alkyl-sulfinyl oder $C_1$-$C_4$-Alkylsulfonyl, für gegebenenfalls halogen-substituiertes $C_1$-$C_8$-Alkyl, $C_2$-$C_6$-Alkenyl oder $C_2$-$C_6$-Alkinyl, für Cyano-$C_1$-$C_4$-alkyl, $C_1$-$C_4$-Alkoxy-$C_1$-$C_2$-alkyl, Phenoxy- und Phenylthio-methyl, Benzyloxy- und Benzylthio-methyl, $C_1$-$C_4$-Alkylthio-$C_1$-$C_2$-alkyl, $C_1$-$C_4$-Alkyl- und Phenyl-sulfinyl-$C_1$-$C_2$-alkyl, $C_1$-$C_4$-Alkyl- und Phenyl-sulfonyl-$C_1$-$C_2$-alkyl, Carboxy-$C_1$-$C_2$-alkyl, $C_1$-$C_4$-Alkoxy-carbonyl-$C_1$-$C_2$-alkyl, $C_1$-$C_4$-Alkyl-amino-carbonyl-$C_1$-$C_2$-alkyl, Di-$C_1$-$C_4$-alkylamino-carbonyl-$C_1$-$C_2$-alkyl, Phenylaminocarbonylalkyl oder $C_3$-$C_{12}$-Cycloalkyl stehen.

Man erhält die neuen Azolyloxyessigsäureamide der Formel I, wenn man Hydroxyzssigäureamide der Formel II

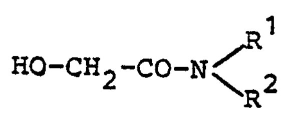

(II)

in welcher $R^1$ und $R^2$ die oben angegebene Bedeutung haben, mit Halogenazolen der Formel III

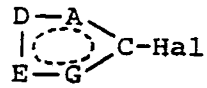 (III)

worin

A, D, E und G die oben angegebene Bedeutung haben und Hal für Chlor, Brom oder Jod steht, gegebenenfalls in Gegenwart eines Säureakzeptors und gegebenenfalls unter Verwendung eines Verdünnungs-mittels umsetzt.

Einzelne der neuen Verbindungen der Formel I können zwar auch auf andere Weise synthetisiert werden, beispielsweise aus den entsprechenden Hydroxyazolen (bzw. den dazu tautomeren Azolonen) und Halogenessigsäureamiden, oder aus entsprechenden Azolyloxyessigsäureestern und Aminen ; jedoch ist die Anwendungsbreite dieser Methoden relativ gering.

Die neuen Azolyloxyessigsäureamide der Formel I zeichnen sich durch starke herbizide Wirksamkeit aus.

Überraschenderweise zeigen die neuen Azolyloxyessigsäureamide eine wesentlich bessere und andersartige herbizide Wirkung als die aus dem Stand der Technik bekannten Phenoxycarbonsäureamide. Insbesondere überrascht die Tatsache, daß die erfindungsgemäßen Verbindungen bei guter Verträglichkeit gegenüber Nutzpflanzen neben ihrer hohen Wirkung gegen dikotyle Unkräuter auch sehr gute Wirkung gegen Ungräser zeigen, während konstitutionell ähnliche Phenoxy-alkancarbonsäurederivate, wie z.B. 2,4 Dichlorphenoxy-essigsäureamid, nur geringe Wirkung gegen Gramineen aufweisen.

Die Erfindung betrifft bevorzugt Verbindungen der Formel I, in welcher

$R^1$ für Wasserstoff, $C_1$-$C_5$-Alkyl, Cyanoethyl, $C_1$-$C_4$-Alkoxy-ethyl, Allyl, Propargyl, 1-Methyl-propargyl, 1,1-Dimethyl-propargyl, Cyclopentyl, Cyclohexyl, Phenyl oder Benzyl steht,

$R^2$ für $C_1$-$C_5$-Alkyl, Cyanoethyl, $C_1$-$C_4$-Alkoxy-ethyl, Allyl, Propargyl, 1-Methyl-propargyl, 1,1-Dimethyl-propargyl, Cyclopentyl, Cyclohexyl, Benzyl, Naphthyl oder Phenyl, welches gegebenenfalls durch 1 bis 3 Reste (Methyl, Fluor, Chlor, Trifluormethyl, Cyano, Nitro oder Methoxy) substituiert ist, steht, oder worin die Reste $R^1$ und $R^2$ zusammen mit dem Stickstoffatom, an das sie gebunden sind, für Pyrrolidyl, Monoalkyl- oder Dialkyl-pyrrolidyl mit 1 bis 3 Kohlenstoffatomen je Alkylgruppe, Morpholinyl oder Dialkylmorpholinyl mit 1 bis 3 Kohlenstoffatomen je Alkylgruppe, Piperidyl, Monoalkyl-, Dialkyl- oder Trialkyl-piperidyl mit 1 bis 3 Kohlenstoffatomen je Alkylgruppe, für 4,4-Dialkoxy-piperidyl mit 1 bis 3 Kohlenstoffatomen je Alkoxygruppe, für spirosubstituiertes Piperidyl der Formel

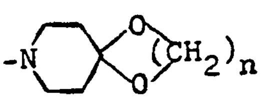

worin n für 2 oder 3 steht, für Perhydroazepinyl (Hexamethylenimino-Rest), Trimethyl-perhydroazepinyl, für den Heptamethylenimino-Rest, für den Dodekamethylenimino-Rest, für 1,2,3,4-Tetrahydroindolyl, für Monoalkyl-, Dialkyl- oder Trialkyl-1,2,3,4-tetrahydroindolyl mit bis zu 3 Kohlenstoffatomen je Alkylgruppe, für Perhydroindolyl, Monoalkyl-, Dialkyl- oder Trialkyl-perhydroindolyl mit 1 bis 3 Kohlenstoffatomen je Alkylgruppe, 1,2,3,4-Tetrahydrochinolyl oder 1,2,3,4-Tetrahydro-iso-chinolyl, Monoalkyl-, Dialkyl- oder Trialkyl-1,2,3,4-tetrahydrochinolyl oder -iso-chinolyl mit 1 bis 3 Kohlenstoffatomen je Alkylgruppe, für Perhydrochinolyl oder Perhydro-iso-chinolyl, Monoalkyl-, Dialkyl- oder Trialkylperhydrochinolyl oder -perhydroisochinolyl mit 1 bis 3 Kohlenstoffatomen je Alkylgruppe, für Perhydrothiazolyl, für Perhydrooxazolyl, für Perhydrooxazinyl, für den Rest

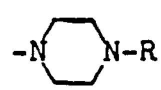

worin R für $C_1$-$C_4$-Alkyl, für gegebenenfalls durch $C_1$-$C_2$-Alkyl, $C_1$-$C_2$-Alkoxy, Fluor, Chlor, Brom, Trifluormethyl und/oder Nitro substituiertes Phenyl, für Benzyl oder Phenylethyl stehen oder worin die Reste $R^1$ und $R^2$ zusammen mit dem Stickstoffatom, an das sie gebunden sind, für den Rest

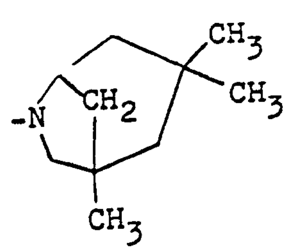

stehen, und in welcher ferner der Rest

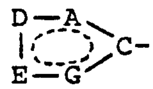

für einen der nachstehenden Azolylreste steht :

(Ia) 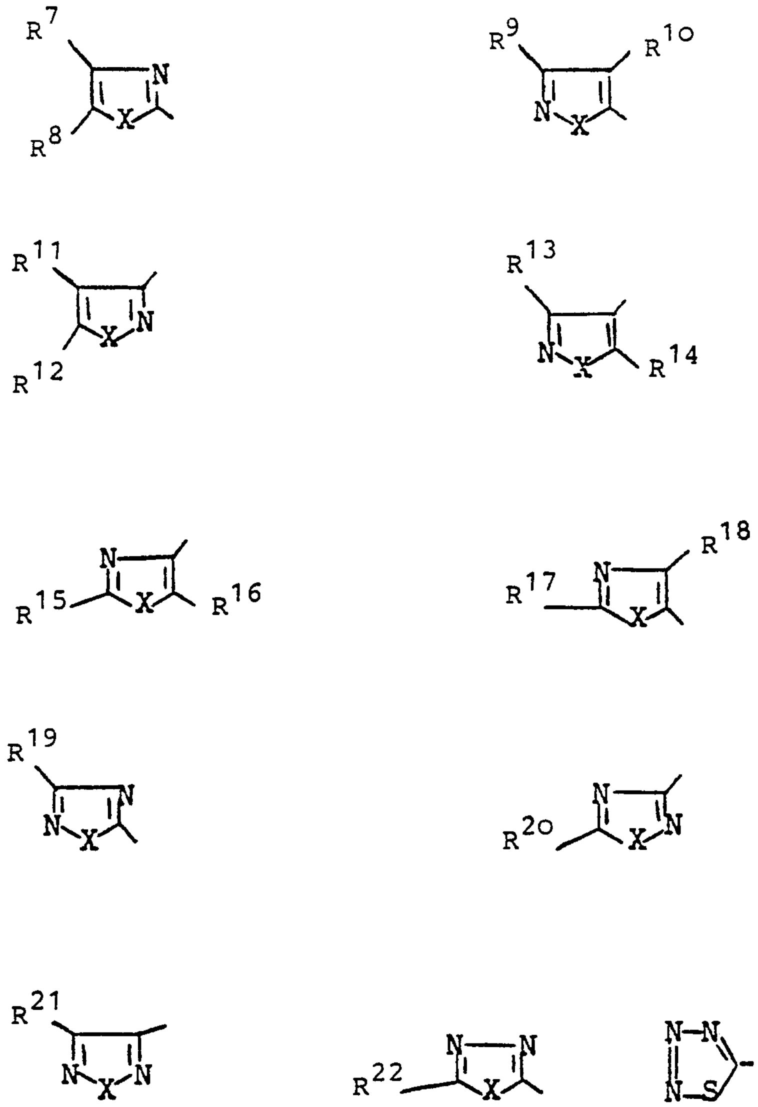

worin

X jeweils für Sauerstoff oder Schwefel steht und die Reste $R^7$ bis $R^{22}$ welche gleich oder verschieden sein können, einzeln für

Wasserstoff, Brom, Chlor, Nitro, Cyano, $C_1$-$C_3$-Alkylcarbonyl, $C_1$-$C_3$-Alkoxycarbonyl, gegebenenfalls durch Fluor, Chlor oder Brom, Methyl, Halogenmethyl, Methoxy, Nitro und/oder Cyano 1- oder 2fach substituiertes Phenyl, für

Phenoxy oder Phenylthio, für $C_1$-$C_4$-Alkylthio oder $C_1$-$C_4$-Alkoxy, für $C_1$-$C_4$-Alkylsulfinyl oder $C_1$-$C_4$-Alkylsulfonyl, für $C_1$-$C_4$-Alkyl, Trifluormethyl, Trichlormethyl, Cyano-$C_1$-$C_4$-Alkyl, $C_2$-$C_4$-Alkenyl, Benzyloxymethyl, $C_1$-$C_3$-Alkyl-amino, N-$C_1$-$C_3$-Alkyl-N-$C_1$-$C_4$-alkyl-aminocarbonyl, Benzylthio oder Phenoxymethyl stehen.

Verwendet man beispielsweise 2,4,5-Trichlor-thiazol-(1,3) und Hydroxyessigsäure-piperidid als Ausgangsstoffe, so kann der Reaktionsablauf nach dem erfindungsgemäßen Verfahren durch folgendes Formelschema wiedergegeben werden :

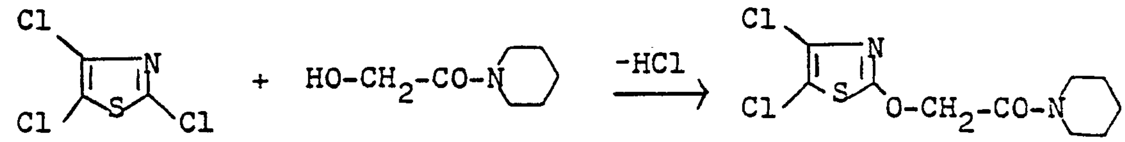


Die als Ausgangsstoffe zu verwendenden Hydroxyessigsäureamide sind durch Formel II definiert. In dieser Formel stehen $R^1$ und $R^2$ vorzugsweise für diejenigen Reste, welche bereits im Rahmen der Substituentendefinitionen der Formel I als bevorzugt genannt worden sind.

Als Ausgangsstoffe der Formel II seien beispielsweise genannt :

Hydroxyessigsäure-methylamid, -ethylamid, -n-propylamid, -iso-propylamid, -n-butylamid, -iso-butylamid -dimethylamid, -diethylamid, -di-n-propyl-amid, -di-iso-propylamid, -N-methyl-N-iso-propyl-amid, -N-methyl-N-iso-butyl-amid, -N-methyl-N-sek.-butyl-amid, -N-propyl-N-sek.-butyl-amid, -N-methyl-N-(2-cyano-ethyl)-amid, -di-(2-methoxy-ethyl)-amid, -di-allyl-amid, -N-methyl-N-propargyl-amid, -N-methyl-N-(1-methyl-propargyl)-amid, -dipropargyl-amid, -cyclopentyl-amid, -N-methyl-N-cyclopentyl-amid, -cyclohexyl-amid, -N-methyl-N-cyclohexyl-amid, -anilid, -2-nitro-, -3-nitro- und 4-nitro-phenyl-amid, -2-chlor-, 3-chlor- und 4-chlor-phenyl-amid, -2,4-dichlor-, 2,5-dichlor-, 3,4-dichlor- und 3,5-dichlor-phenyl-amid, -2-methyl-, -3-methyl- und -4-methyl-phenyl-amid, -N-methyl-anilid, -N-methyl-N-(2-nitro-phenyl)-, -N-methyl-N-(3-nitro-phenyl)-, -N-methyl-N-(4-nitro-phenyl)-amid, -N-methyl-N-(2-chlor-phenyl)-, -N-methyl-N-(3-chlor-phenyl)-, -N-methyl-N-(4-chlor-phenyl)-amid, -N-methyl-N-(3-nitro-6-methyl-phenyl)-amid, -N-ethylanilid, -N-ethyl-N-(2-nitro-phenyl)-, -N-ethyl-N-(3-nitro-phenyl)-, -N-ethyl-N-(4-nitro-phenyl)-amid, -N-ethyl-N-(2-chlor-phenyl)-, -N-ethyl-N-(3-chlor-phenyl)-, -N-ethyl-N-(4-chlor-phenyl)-amid, -N-ethyl-N-(3-nitro-6-methyl-phenyl)-amid, -N-propyl-anilid, -N-propyl-N-(2-nitro-phenyl)-, -N-propyl-N-(3-nitro-phenyl)-, -N-propyl-N-(4-nitro-phenyl)-amid, -N-propyl-N-(2-chlor-phenyl)-, -N-propyl-N-(3-chlor-phenyl)-, -N-propyl-N-(4-chlor-phenyl)-amid, -N-propyl-N-(2-methyl-phenyl)-, -N-propyl-N-(3-methyl-phenyl)-, -N-propyl-N-(4-methyl-phenyl)-amid, -N-propyl-N-(3-nitro-6-methyl-phenyl)-amid, -N-butyl-anilid, -N-butyl-N-(2-nitro-phenyl)-, -N-butyl-N-(3-nitro-phenyl)-, -N-butyl-N-(4-nitro-phenyl)-amid, -N-butyl-N-(2-chlorphenyl)-, -N-butyl-N-(3-chlor-phenyl)-, -N-butyl-N-(4-chlor-phenyl)-amid, -N-butyl-N-(2-methyl-phenyl)-, -N-butyl-N-(3-methyl-phenyl)-, -N-butyl-N-(4-methyl-phenyl)-amid, -N-butyl-N-(3-nitro-6-methyl-phenyl)-amid, -N-isobutyl-anilid, -N-iso-butyl-N-(2-nitro-phenyl)-, -N-iso-butyl-N-(3-nitro-phenyl)-, -N-iso-butyl-N-(4-nitro-phenyl)-amid, -N-iso-butyl-N-(2-chlor-phenyl)-, -N-isobutyl-N-(3-chlor-phenyl)-, -N-iso-butyl-N-(4-chlor-phenyl)-amid, -N-iso-butyl-N-(2-methyl-phenyl)-, -N-iso-butyl-N-(3-methyl-phenyl)-, -N-iso-butyl-N-(4-methyl-phenyl)-amid, -N-iso-butyl-N-(3-nitro-6-methyl-phenyl)-amid, -naphth(1)ylamid, -naphth(2)ylamid, -N-methyl-N-naphth(1)-ylamid, -N-methyl-N-naphth(2)ylamid, -N-ethyl-N-naphth(1)ylamid, -N-ethyl-N-naphth(2)ylamid, -N-n-propyl-N-naphth(2)ylamid, -N-iso-propyl-N-naphth(2)ylamid, -N-n-butyl-N-naphth(2)ylamid, -N-iso-butyl-N-naphth(2)ylamid, -benzylamid, -dibenzylamid, -N-methyl-N-benzylamid, -N-ethyl-N-benzylamid, -N-propyl-N-benzylamid, -N-butyl-N-benzylamid, -pyrrolidid, -2-methyl-pyrrolidid, -morpholid, -piperidid, -2-methyl-piperidid, -4-methyl-piperidid, -2,4-dimethyl-piperidid, -2,4,6-trimethyl-piperidid, -2-ethyl-piperidid, -4-ethyl-piperidid, -2,4-diethyl-piperidid, -2,4,6-triethyl-piperidid, -2-methyl-4-ethyl-piperidid, -2-ethyl-4-methyl-piperidid, -2-methyl-5-ethyl-piperidid, -2-ethyl-5-methyl-piperidid, -2-methyl-6-ethyl-piperidid, -1,2,3,4-tetrahydroindolid, -2-methyl-1,2,3,4-tetrahydroindolid, -perhydroindolid, -2-methyl-perhydroindolid, -2,2-dimethyl-perhydroindolid, -1,2,3,4-tetrahydrochinolid, -2-methyl-1,2,3,4-tetrahydrochinolid, -perhydrochinolid, -2-methyl-perhydrochinolid, -1,2,3,4-tetrahydro-isochinolid und -perhydroisochinolid.

Die Hydroxyessigsäureamide der Formel II sind teilweise bekannt (vgl. US-A 3 399 988 ; DE-A 2 201 432 und DE-A 2 647 481). Sie können, wie im nachstehenden Schema skizziert, ausgehend von Chloressigsäurechloriden hergestellt werden :

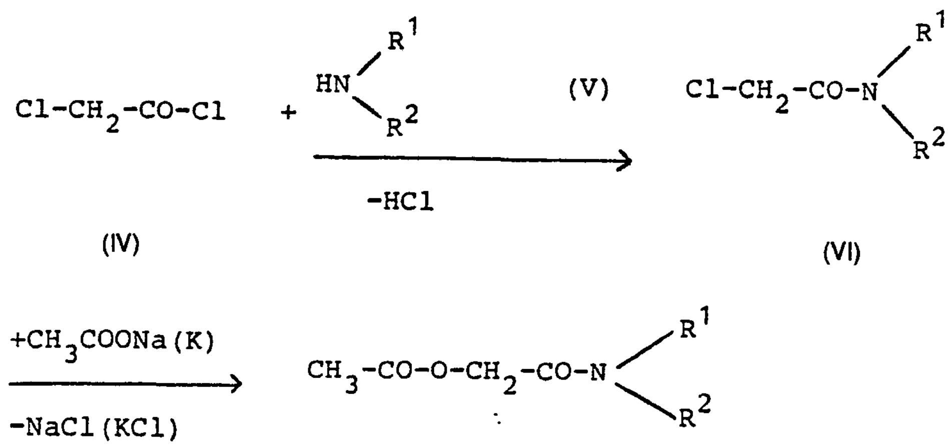


(IV)

(VI)

(VII)

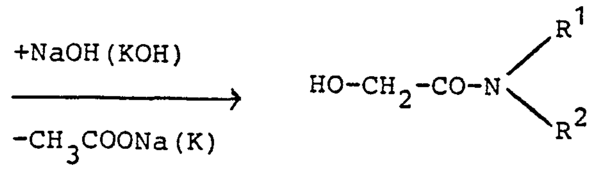


(II)

Hierzu wird zunächst das literaturbekannte Chloressigsäurechlorid der Formel IV mit Aminen der Formel V — wobei $R^1$ und $R^2$ die oben angegebene Bedeutung haben — gegebenenfalls in Gegenwart eines Säurebindemittels wie z.B. Triäthylamin und gegebenenfalls unter Verwendung eines inerten Verdünnungsmittels, wie z.B. 1,2-Dichlorethan, bei Temperaturen zwischen – 20 und 100 °C, vorzugsweise zwischen – 10 und 50 °C in die entsprechenden Chloressigsäureamide der Formel VI überführt. Die Aufarbeitung dieser Produkte erfolgt nach üblichen Methoden durch Waschen mit Wasser, Trocknen der organischen Phase und Abdestillieren des Lösungsmittels.

Die Verbindungen der Formel VI werden mit Natrium- oder Kalium-acetat, gegebenenfalls unter Verwendung eines Verdünnungsmittels wie z.B. Essigsäure oder Dimethylsulfoxid, bei Temperaturen zwischen 20 und 150 °C, vorzugsweise zwischen 50 und 120 °C, zu den entsprechenden Acetoxyessigsäureamiden der Formel VII umgesetzt. Soweit die Produkte hierbei kristallin anfallen, werden sie durch Absaugen isoliert. Andernfalls erfolgt die Aufarbeitung nach üblichen Methoden, beispielsweise durch Abdestillieren des Lösungsmittels im Vakuum, Aufnehmen des Rückstandes in Methylenchlorid, Waschen mit Wasser und Abdestillieren des Lösungsmittels.

Durch Umsetzung mit wässrig-alkoholischer Natronlauge oder Kalilauge bei Temperaturen zwischen 0 und 100 °C, vorzugsweise zwischen 10 und 50 °C, können die Verbindungen der Formel VII zu den Verbindungen der Formel II entacyliert werden. Zur Isolierung der Produkte werden die Lösungsmittel in Vakuum abdestilliert, der Rückstand wird mit einem organischen Lösungsmittel wie z.B. Methylenchlorid oder Essigester extrahiert, die Lösung getrocknet und das Lösungsmittel abdestilliert.

Die weiter als Ausgangsstoffe zu verwendenden Halogenazole sind durch Formel III definiert. In dieser Formel steht der Rest

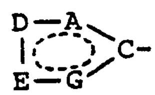

vorzugsweise für diejenigen Reste, welche bereits im Rahmen der Substituentendefinitionen der Formel I als bevorzugt genannt worden sind und Hal steht vorzugsweise für Chlor oder Brom.

Als Ausgangsstoffe der Formel III seien beispielsweise genannt :

2-Chlor- und 2-Brom-oxazol und -thiazol, 2,4-Dichlor-, 2,5-Dichlor- und 2,4,5-Trichlor-oxazol und -thiazol, 4-Methyl-, 5-Methyl-, 4-tert.-Butyl-, 4,5-Dimethyl-, 4-Methyl-5-chlor-, 5-Methyl-4-chlor-, 4-Methyl-5-methoxycarbonyl-, 4-Methyl-5-ethoxycarbonyl-, 4-Methyl-5-isopropoxycarbonyl-, 4-Methyl-5-acetyl-, 5-Phenyl-, 4,5-Diphenyl-, 4-Chlor-5-phenyl-, 4-Chlor-5-(3,4-dichlor-phenyl)- und 4-Methyl-5-phenylthio-2-chlor-oxazol, -2-brom-oxazol, -2-chlor-thiazol und -2-brom-thiazol ; 3-tert.-Butyl-4-cyano-, 3-But-3-en-1-yl-, 3,4-Bis-ethoxycarbonyl-, 3-Phenyl-, 3-Ethyl-4-phenyl-5-chlor-isoxazol, -5-chlor-isothiazol, -5-brom-isoxazol und -5-brom-isothiazol ; 3,5-Bis-ethoxycarbonyl-4-chlor- und 3,5-Bis-ethoxycarbonyl-4-brom-isoxazol und -isothiazol ; 3,5-Dichlor-1,2,4-oxadiazol, 3-Methyl-, 3-Ethyl-, 3-n-Propyl-, 3-iso-Propyl-, 3-tert.-Butyl-, 3-Trifluormethyl-, 3-Trichlormethyl-, 3-Methylthio-, 3-Methylsulfinyl-, 3-Methylsulfonyl-5-chlor-1,2,4-thiadiazol und -5-brom-1,2,4-thiadiazol ; 4-Methyl-5-cyano-2-chloro- und 4-Phenyl-5-cyano-2-chloro-thiazol ; 4-Methyl-, 4-Ethyl-, 4-n-Propyl- und 4-iso-Propyl-3-chlor-1,2,5-thiadiazol und -3-brom-1,2,5-thiadiazol ; 2-Chlor- und 2-Brom-1,3,4-oxadiazol, 2-Chlor- und 2-Brom-1,3,4-thiadiazol, 5-Methyl-, 5-Ethyl-, 5-n-Propyl-, 5-iso-Propyl-, 5-tert.Butyl-, 5-Brom-, 5-Methylsulfinyl-, 5-Ethylsulfinyl-, 5-Propylsulfinyl-, 5-Methylsulfonyl-, 5-Ethylsulfonyl-, 5-Propyl-sulfonyl-, 5-Methoxycarbonyl-, 5-Ethoxy-carbonyl-, 5-(1-Cyano-2-methylpropyl)-, 5-Benzyloxymethyl-, 5-Acetylamino-, 5-Nitro, 5-Propylthio-, 5-Trifluormethyl-, 5-Trichlormethyl-, 5-Methylamino- und 5-(N-Methyl-N-tert.-butylcarbonyl-amino)-2-chlor-1,3,4-oxadiazol, -2-brom-1,3,4-oxadiazol, -2-chlor-1,3,4-thiadiazol und -2-brom-1,3,4-thiadiazol.

Halogenazole der Formel III sind bekannt (vgl. Elderfield, Heterocyclic Compounds Vol. *5* (1957), S. 298 und S. 452 ; Vol. *7* (1961), S. 463 und S. 541 ; Weissberger, The Chemistry of Heterocyclic Compounds, (a) « Five-Membered Heterocyclic Compounds with Nitrogen and Sulfur or Nitrogen, Sulfur and Oxygen » (1952), S. 35 und S. 81, (b) « Five- and Six-Membered Compounds with Nitrogen and Oxygen » (1962), S. 5, S. 245 und S. 263 ; Advances in Heterocyclic Chemistry, Vol. *5* (1965), S. 119 ; Vol. *7* (1966), S. 183 ; Vol. *9* (1968), S. 107, S. 165 und S. 183 ; Vol. *17* (1974), S. 99 und Vol. *20* (1976), S. 65 ;

Synthesis *1978*, 803 ; Tetrahedron Letters *1968*, 829 ; Chem. Ber. *89* (1956), 1534 ; *90* (1957), 182 ; *92* (1959) 1928 ; J. Org. Chem. *27* (1962), 2589, DE-A 22 13 865).

Einige der als Ausgangsstoffe zu verwendenden Halogenazole sind noch nicht in der Literatur beschrieben. Verschiedene Halogenazole erhält man beispielsweise, indem man entsprechende Aminoazole mit Halogenwasserstoffsäuren in Wasser löst und unter Eiskühlung mit Natriumnitrit umsetzt, die Mischungen einige Stunden bei Temperaturen zwischen 10 und 50 °C rührt, die Produkte mit Toluol extrahiert und nach Waschen und Trocknen die organische Phase destillativ aufarbeitet (vgl. DE-A 2 144 326).

Das erfindungsgemäße Verfahren zur Herstellung der neuen Azolyloxyessigsäureamide I wird vorzugsweise unter Verwendung geeigneter Lösungs- oder Verdünnungsmittel durchgeführt. Als solche kommen praktisch alle inerten organischen Solventien infrage. Hierzu gehören insbesondere Alkohole, wie Methanol, Ethanol, n- und iso-Propanol, n-, iso-, sek.- und tert.-Butanol, Ether wie Diethylether, Dibutylether, Tetrahydrofuran und Dioxan, Ketone wie Aceton, Methylethylketon, Methylisopropylketon und Methylisobutylketon, Nitrile wie Acetonitril und Propionsäurenitril, sowie die hochpolaren Lösungsmittel Dimethylformamid, Dimethylsulfoxid, Sulfolan und Hexamethylphosphorsäuretriamid.

Als Säureakzeptoren können bei dem erfindungsgemäßen Verfahren praktisch alle üblicherweise verwendbaren Säurebindemittel eingesetzt werden : hierzu gehören insbesondere Alkali- und Erdalkalihydroxide bzw. -oxide wie Natrium- und Kaliumhydroxid sowie Calcium-oxid oder Calcium-hydroxid, Alkali- und Erdalkali-carbonate wie Natrium-, Kalium- und Calciumcarbonat, Alkalialkoholate wie Natrium-methylat, -ethylat und -tert.-butylat, Kaliummethylat, -ethylat und -tert.-butylat, ferner aliphatische, aromatische oder heterocyclische Amine wie Triethylamin, Dimethylanilin, Dimethylbenzylamin, Pyridin, Diazabicyclooctan und Diazabicycloundecen.

Die Reaktionstemperatur kann innerhalb eines größeren Bereichs variiert werden. Im allgemeinen arbeitet man zwischen − 50 und + 150 °C, vorzugsweise bei − 20 bis + 100 °C.

Das erfindungsgemäße Verfahren wird im allgemeinen bei Normaldruck durchgeführt.

Bei der Durchführung des erfindungsgemäßen Verfahrens setzt man auf 1 Mol Halogenazol der Formel III 1,0 bis 1,5 Mol Hydroxyessigsäureamid der Formel II ein. Die Umsetzung wird im allgemeinen in einem geeigneten Verdünnungsmittel durchgeführt und das Reaktionsgemisch wird bei der erforderlichen Temperatur mehrere Stunden gerührt.

Die Isolierung der Produkte erfolgt nach üblichen Methoden : Man destilliert gegebenenfalls einen Teil des Verdünnungsmittels unter vermindertem Druck ab und gießt den Rest der Reaktionsmischung in Wasser. Soweit die Produkte hierbei kristallin anfallen, werden sie durch Absaugen isoliert. Andernfalls werden die organischen Produkte mit einem mit Wasser nicht mischbaren Lösungsmittel wie z.B. Toluol oder Methylenchlorid extrahiert ; nach Waschen und Trocknen wird dann von der organischen Phase das Lösungsmittel im Vakuum abdestilliert. Die zurückbleibenden Produkte werden durch ihren Schmelzpunkt bzw. ihren Brechungsindex charakterisiert.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden :

Dikotyle Unkräuter der Gattungen : Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen : Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen : Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen : Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen insbesondere neben einer sehr guten Wirkung gegen grasartige Unkräuter auch eine gute herbizide Wirkung bei breitblättigten Unkräutern. Ein selektiver

Einsatz der erfindungsgemäßen Wirkstoffe ist in verschiedenen Kulturen möglich, z.B. in Rüben, Sojabohnen, Baumwolle, Reis und anderen Getreidearten. Dabei sind einzelne Wirkstoffe als Selektivherbizide in Rüben, Baumwolle und Getreide besonders geeignet.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermittels und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hoch disperse Kieselsäure, Alminiumoxid und Silicate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z.B. nichtionogene und antionische Emulgatoren, wie Polyoxyethylen-Fettsäure-Ester, Polyoxyethylen-Fettalkohol-Ether, z.B. Alkylaryl-polyglykol-ether, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummi arabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen oder Stäuben.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im preemergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,01 und 10 kg Wirkstoff pro ha, vorzugsweise zwischen 0,1 und 5 kg/ha.

Die erfindungsgemäßen Wirkstoffe weisen zum Teil bei bestimmten Anwendungskonzentrationen auch eine wachstumsregulierende Wirkung auf.

Die nachfolgenden Beispiele dienen zur Erläuterung der Erfindung.

## Herstellungsbeispiele

### Beispiel 1

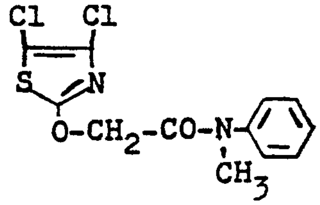

14,8 g (0,09 Mol) Hydroxyessigsäure-N-methylanilid werden in 100 ml Isopropanol gelöst. Die Lösung wird mit 5,6 g gepulvertem Kaliumhydroxyd verrührt und auf – 15 °C gekühlt. Bei – 15 °C werden in einer Stunde 13,1 g (0,07 Mol) 2,4,5-Trichlorthiazol hinzugetropft. Man läßt 3 Stunden bei – 10 °C rühren und anschließend die Temperatur auf 0 °C ansteigen, rührt 2 Stunden bei 0 °C und anschließend 10 Stunden bei Raumtemperatur. Dann werden 3/4 des Lösungsmittels abdestilliert, der Rückstand wird auf Wasser gegossen und der ausfallende Festkörper abgesaugt und getrocknet. Die Ausbeute beträgt 18 g (81 % der Theorie) ; Schmelzpunkt : 82 °C ; O-(4,5-Dichlor-1,3-thiazol-2-yl)-oxyessigsäure-N-methylanilid.

Beispiel 2

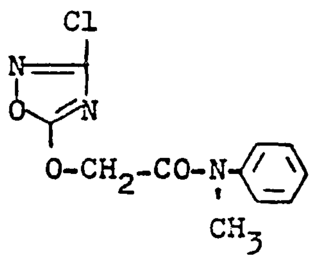

9 g (0,05 Mol) Hydroxyessigsäure-N-methylanilid werden in 100 ml Acetonitril gelöst. Dann werden 7,6 g Kaliumcarbonat hinzugegeben. Bei – 10 °C werden 6,9 g 3,5-Dichlor-1,2,4-oxadiazol langsam zugetropft. Dann wird noch 2 Stunden bei – 10 °C, 3 Stunden bei 0-5 °C und 20 Stunden bei Raumtemperatur nachgerührt. Dann wird das Acetonitril im Vakuum bis auf 1/4 abdestilliert, der Rückstand auf Wasser gegossen und das Produkt mit Toluol extrahiert. Nach dem Abdestillieren des Toluols kristallisiert die Substanz. Die Ausbeute beträgt 9 g (70 % der Theorie) ; Schmelzpunkt 73 °C ; O-(3-Chlor-1,2,4-oxadiazol-5-yl)-oxyessigsäure-N-methylanilid.

Beispiel 3

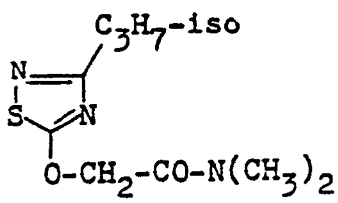

8,6 g (0,082 Mol) Hydroxyessigsäure-dimethylamid werden in 150 ml tert.-Butanol zusammen mit 11,2 g Kalium-tert.-butylat verrührt. Bei 20 °C werden langsam 13,3 g 5-Chlor-3-isopropyl-1,2,4-thiadiazol zugetropft. Man läßt 4 Stunden bei ca. 40 °C rühren. Dann wird das tert.-Butanol im Vakuum zu 3/4 abdestilliert und der Rückstand auf Wasser gegossen. Das ausfallende Öl wird mit Toluol extrahiert. Nach dem Abdestillieren des Toluols bleibt das Produkt als Kristallisat zurück in einer Ausbeute von 9 g (50 % der Theorie) und mit einem Siedepunkt von 67 °C ; O-(3-Isopropyl-1,2,4-thiadiazol-5-yl)-oxyessigsäuredimethylamid.

Beispiel 4

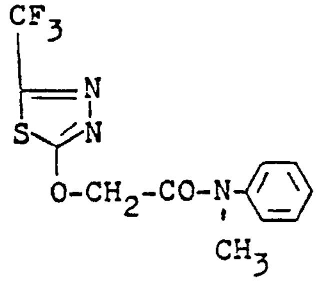

8,7 g (0,05 Mol) 2-Hydroxyessigsäure-N-methylanilid werden zusammen mit 3 g Calciumoxid in 60 ml Dimethylsulfoxid 1 Stunde bei 50 °C gerührt. Dann werden bei 50 °C 11,6 g 5-Brom-2-trifluormethyl-1,3,4-thiadiazol hinzugetropft und 40 Stunden bei 50 °C nachgerührt. Die Lösung wird dann auf 1 l Wasser gegossen und das ausgefallene Öl mit Methylenchlorid extrahiert. Nach dem Abdestillieren des Methylenchlorids bleibt das Produkt als Öl zurück in einer Ausbeute von 10 g mit einem Brechungsindex von $n_D^{21}$ = 1,5404 ; O-(2-Trifluormethyl-1,3,4-thiadiazol-5-y)-oxyessigsäure-N-methylanilid.

Beispiel 5

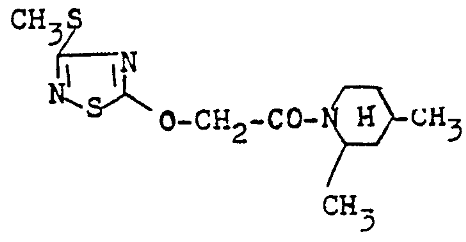

8,3 g (0,05 Mol) 5-Chlor-3-methylthio-1,2,4-thiadiazol werden bei 20-30 °C zu einer Mischung aus 8,5 g (0,05 Mol) Hydroxyessigsäure-2,4-dimethylpiperidid, 3,4 g (0,06 Mol) Kaliumhydroxid-Pulver, 1 g Kupferpulver und 100 ml iso-Propanol gegeben. Die Mischung wird mehrere Stunden gerührt und anschließend mit Wasser verdünnt, dann extrahiert man das Produkt mit Toluol, wäscht die organische Phase mit Wasser, trocknet, filtriert und zieht vom Filtrat das Lösungsmittel im Vakuum ab. Die Ausbeute beträgt 9 g (60 % der Theorie) ; Brechungsindex : $n_D^{23}$ = 1,5460 ; O-(3-Methylthio-1,2,4-thiadiazol-5-yl)-oxyessigsäure-N-(2,4-dimethylpiperidid).

Analog einem der Beispiele 1 bis 5 können die folgenden Verbindungen der Formel Ia

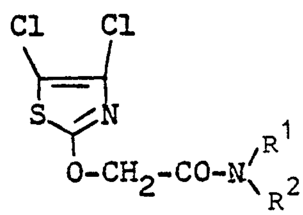

(Ia)

hergestellt werden :

Tabelle I

| Beispiel Nr. | 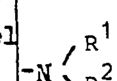 | Physikal. Daten (Brechungsindex: Schmelzpunkt °C) |
|---|---|---|
| 6 | 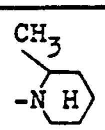 | $n_D^{23}$:1,5449 |
| 7 | 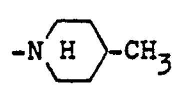 | 38 |
| 8 | $-N(CH_3)_2$ | 85 |
| 9 | $-N(C_2H_5)_2$ | $n_D^{23}$:1,5399 |

| Bei-spiel Nr. | 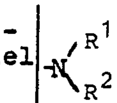 | Physikal. Daten (Brechungsindex; Schmelzpunkt °C) |
|---|---|---|
| 1o | $-N(CH_2-CH=CH_2)_2$ | $n_D^{23}$:1,5418 |
| 11 | 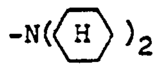 | 127 |
| 12 | 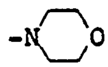 | 125 |
| 13 | 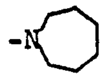 | 62 |
| 14 | 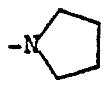 | 63 |
| 15 | $-N(C_3H_7\text{-iso})_2$ | 68 |
| 16 | 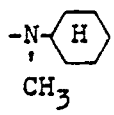 | $n_D^{21}$:1,551o |
| 17 | 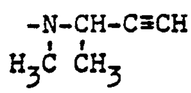 | |
| 18 | $-N(CH_2-CH_2-OCH_3)_2$ | |
| 19 | 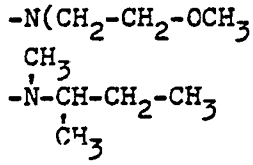 | $n_D^{25}$:1,5357 |
| 2o | 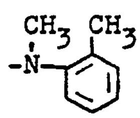 | 88-9o |
| 21 | 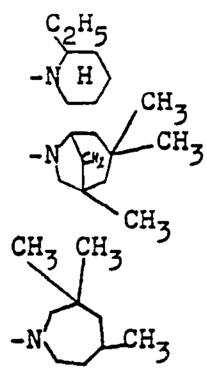 | $n_D^{21}$:1,544o |
| 22 | | 99 |
| 23 | | 9o |
| 24 | 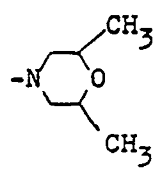 | $n_D^{21}$:1,5549 |
| 25 | 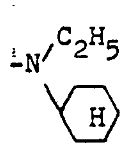 | 103 |

| Beispiel Nr. | $-N<^{R^1}_{R^2}$ | Physikal. Daten (Brechungsindex; Schmelzpunkt °C) |
|---|---|---|
| 26 | 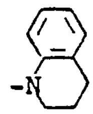 | $n_D^{22}$:1,6083 |
| 27 | 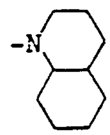 | $n_D^{22}$:1,5570 |
| 28 | 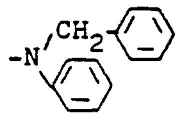 | $n_D^{22}$:1,5972 |
| 29 | 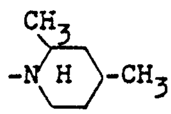 | $n_D^{22}$:1,5432 |
| 30 | 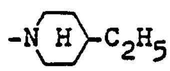 | $n_D^{22}$:1,5500 |

Analog können die folgenden Verbindungen hergestellt werden :

| Beispiel Nr. | Strukturformel | Physikal. Daten (Brechungsindex; Schmelzpunkt °C) |
|---|---|---|
| 31 | 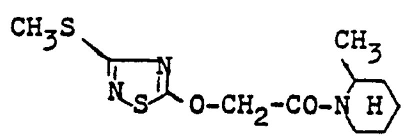 | $n_D^{23}$:1,5560 |
| 32 | 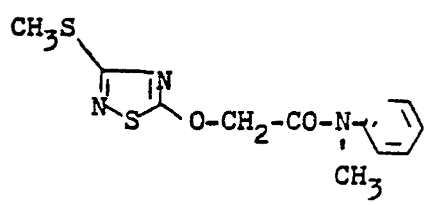 | 98 |
| 33 | 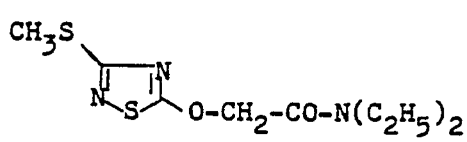 | 68 |
| 34 | 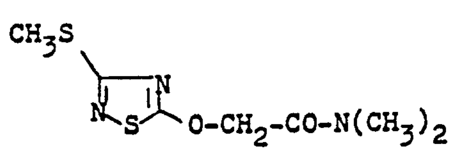 | 150 |

| Beispiel Nr. | Strukturformel | Physikal. Daten (Brechungsindex; Schmelzpunkt °C) |
|---|---|---|
| 35 | 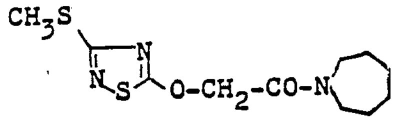 | $n_D^{23}$:1,5440 |
| 36 | 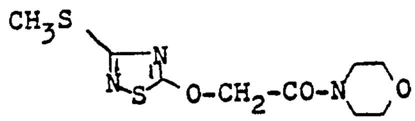 | 156 |
| 37 | 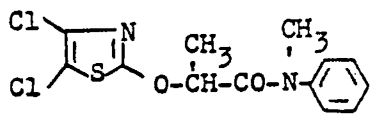 | $n_D^{22}$:1,5584 |
| 38 | 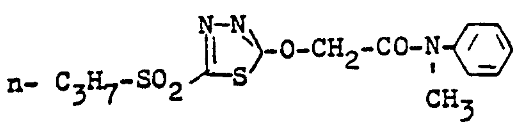 | $n_D^{23}$:1,5675 |
| 39 | 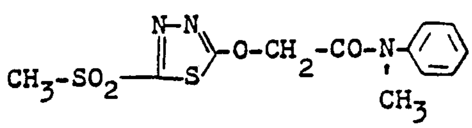 | $n_D^{23}$:1,5534 |
| 40 | 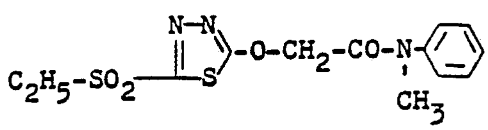 | $n_D^{23}$:1,5321 |
| 41 | 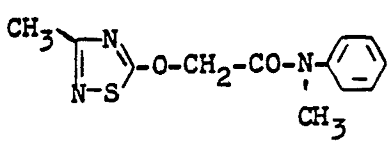 | 64 |
| 42 | 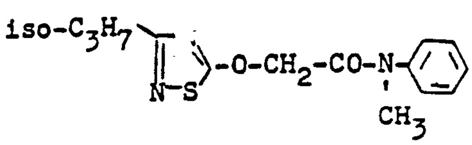 | 120 |
| 43 | 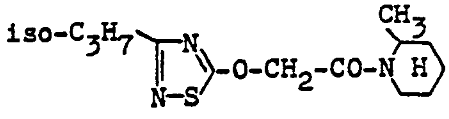 | $n_D^{23}$:1,5135 |

| Beispiel Nr. | Strukturformel | Physikal. Daten (Brechungsindex; Schmelzpunkt °C) |
|---|---|---|
| 44 | 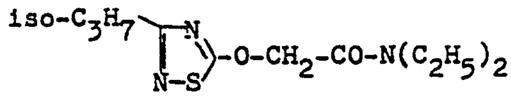 | 44 |
| 45 | 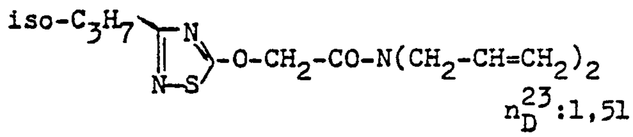 | $n_D^{23}$:1,5142 |
| 46 | 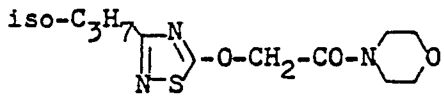 | 72 |
| 47 | 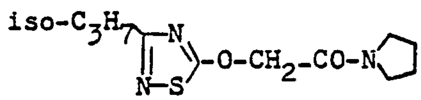 | 54 |
| 48 | 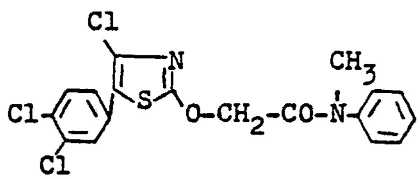 | 118 |
| 49 | 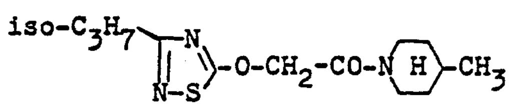 | 56 |
| 50 | 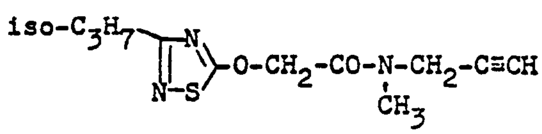 | $n_D^{21}$:1,5230 |
| 51 | 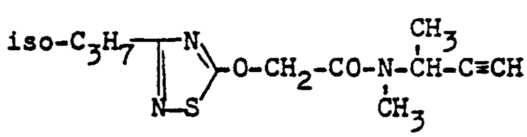 | $n_D^{21}$:1,5155 |
| 52 | 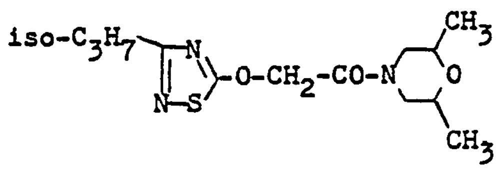 | $n_D^{21}$:1,5140 |
| 53 | 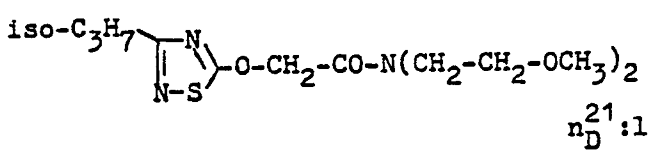 | $n_D^{21}$:1,4987 |

| Beispiel Nr. | Strukturformel | Physikal. Daten (Brechungsindex; Schmelzpunkt °C) |
|---|---|---|
| 54 | 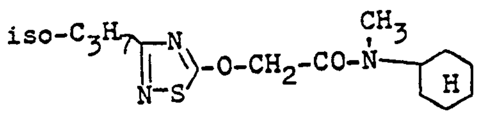 | $n_D^{21}$:1,515o |
| 55 | 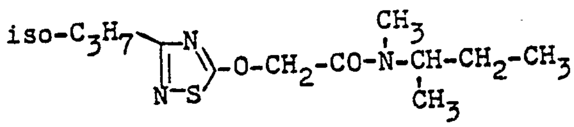 | 46-48 |
| 56 | 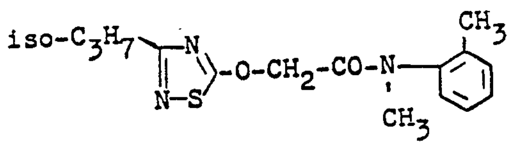 | $n_D^{21}$:1,5428 |
| 57 | 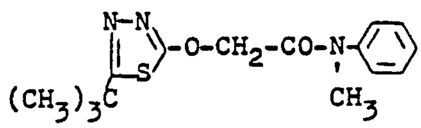 | 85-86 |
| 58 | 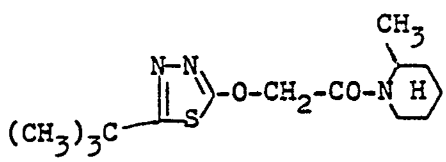 | 84-85 |
| 59 | 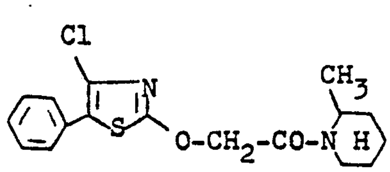 | $n_D^{2o}$:1,5881 |
| 6o | 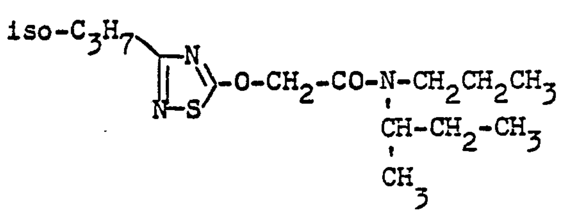 | $n_D^{2o}$:1,4949 |
| 61 | 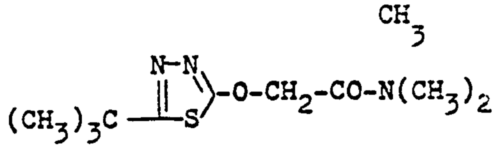 | 78 |
| 62 | 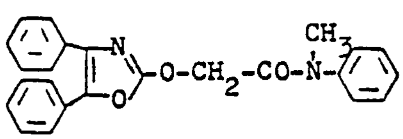 | 1o3 |

| Beispiel Nr. | Strukturformel | Physikal. Daten (Brechungsindex; Schmelzpunkt °C) |
|---|---|---|
| 63 | 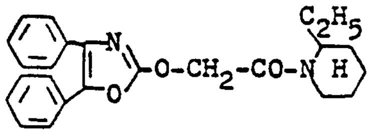 | 116 |
| 64 | 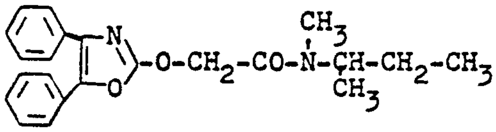 | 134 |
| 65 | 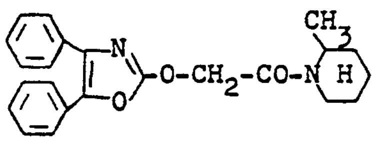 | 156 |
| 66 | 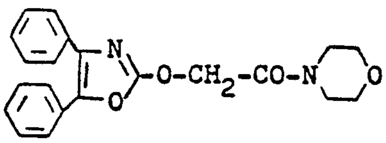 | 11o |
| 67 | 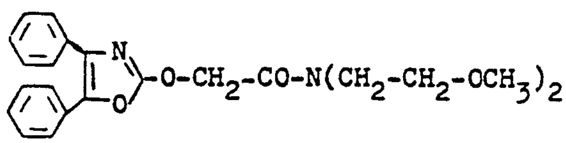 | 92 |
| 68 | 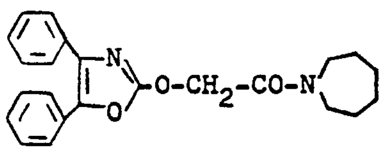 | 143 |
| 69 | 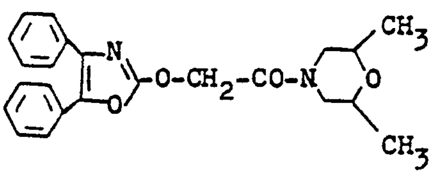 | 93 |
| 7o | 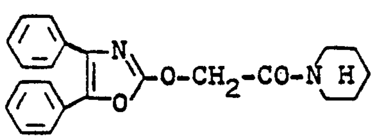 | 143 |
| 71 | 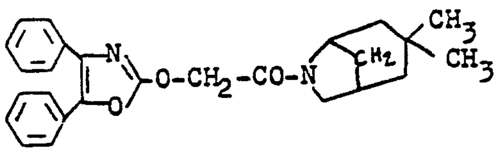 | 133 |
| 72 | 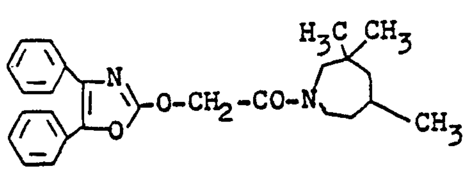 | 9o |

| Beispiel Nr. | Strukturformel | Physikal. Daten (Brechungsindex; Schmelzpunkt °C) |
|---|---|---|
| 73 | 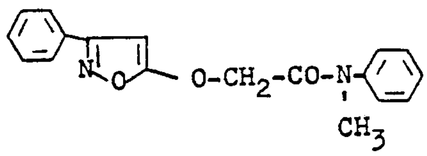 | 138 |
| 74 | 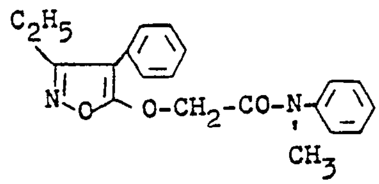 | |
| 75 | 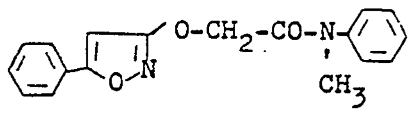 | 11o |
| 76 | 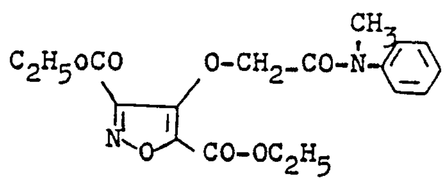 | $n_D^{21}$:1,5334 |
| 77 | 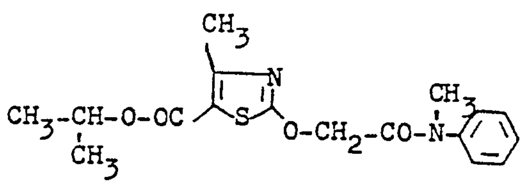 | 115 |
| 78 | 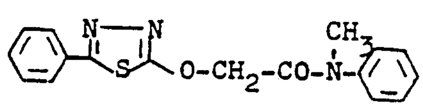 | 114 |
| 79 | 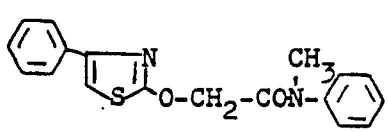 | 1o7 |
| 8o | 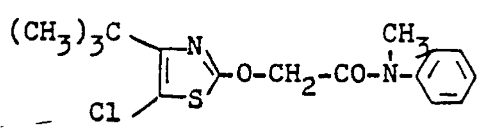 | |
| 81 | 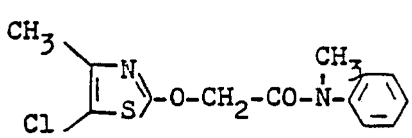 | |

| Beispiel Nr. | Strukturformel | Physikal Daten (Brechungsindex; Schmelzpunkt °C) |
|---|---|---|
| 82 | 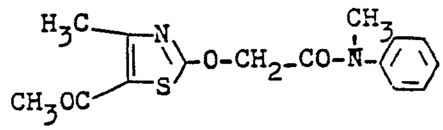 | 160 |
| 83 | 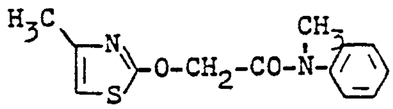 | |
| 84 | 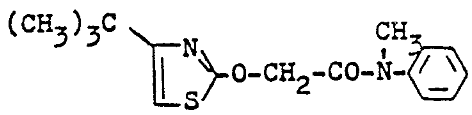 | |
| 85 | 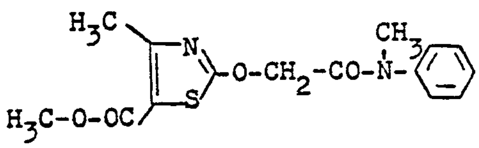 | 142 |
| 86 | 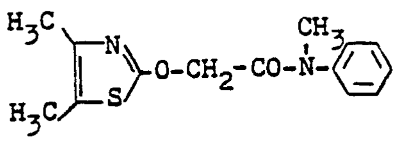 | 148 |
| 87 | 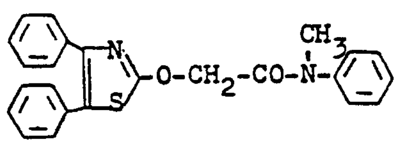 | |
| 88 | 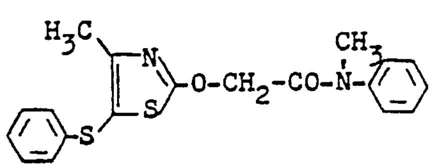 | 63 |
| 89 | 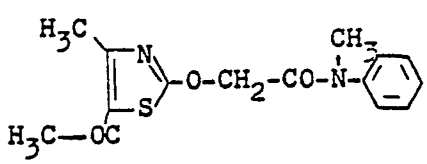 | |

| Beispiel Nr. | Strukturformel | Physikal. Daten (Brechungsindex; Schmelzpunkt oC) |
| --- | --- | --- |
| 9o | 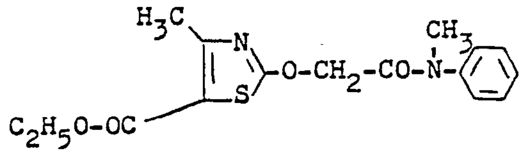 | |
| 91 | 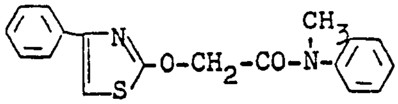 | |
| 92 | 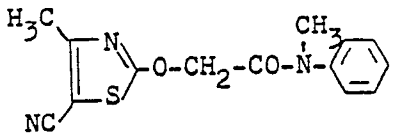 | |
| 93 | 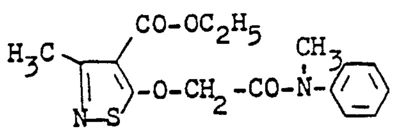 | |
| 94 | 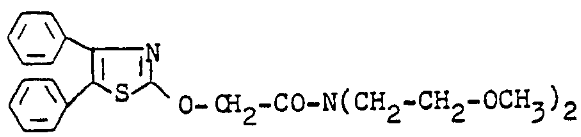 | 82 |
| 95 | 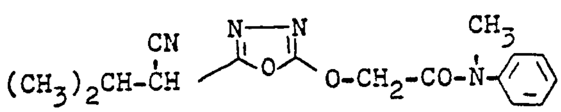 | |
| 96 | 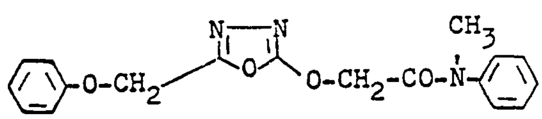 | |
| 97 | 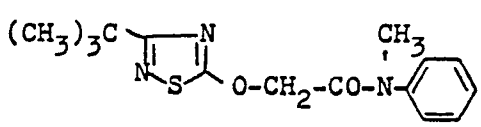 | $n_D^{23}$:1,539o |
| 98 | 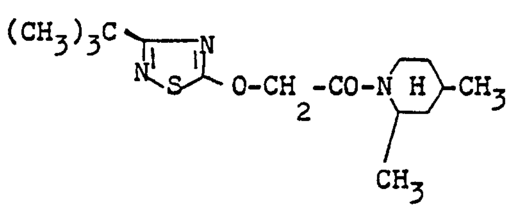 | 78 |

| Bei-spiel Nr. | Strukturformel | Physikal. Daten (Brechungs-index; Schmelz-punkt °C) |
|---|---|---|
| 99 | 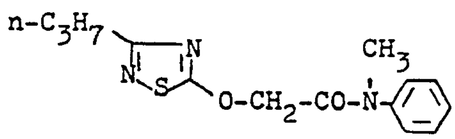 | 47 |
| 1oo | 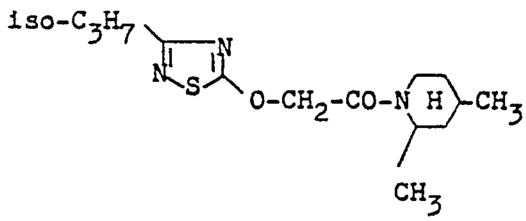 | $n_D^{22}$:1,4791 |
| 1o1 | 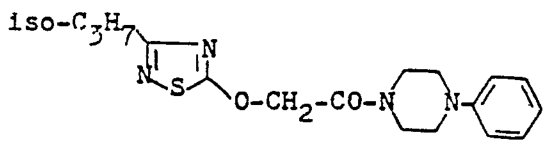 | $n_D^{22}$:1,5632 |
| 1o2 | 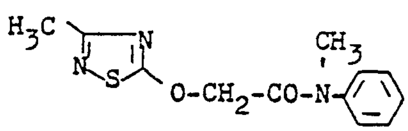 | $n_D^{22}$:1,5442 |
| 1o3 | 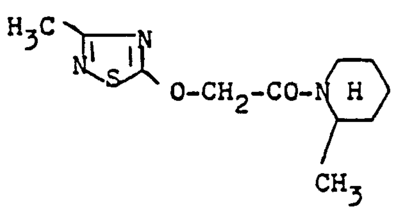 | $n_D^{22}$:1,4885 |
| 1o4 | 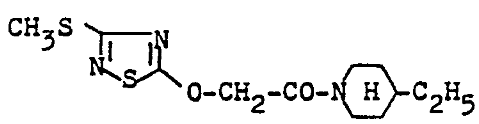 | 87 |
| 1o5 | 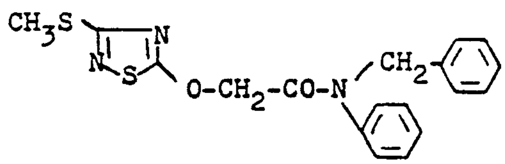 | 84 |
| 1o6 | 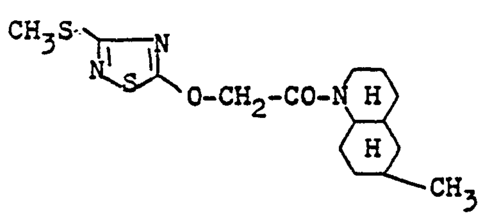 | $n_D^{23}$:1,5547 |

| Bei-spiel Nr. | Strukturformel | Physikal. Daten (Brechungs-index; Schmelz-punkt °C) |
|---|---|---|
| 1o7 | 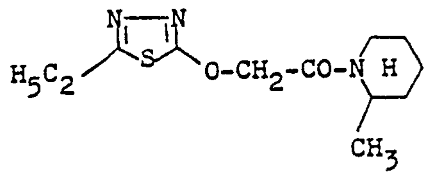 | $n_D^{22}$:1,53oo |
| 1o8 | 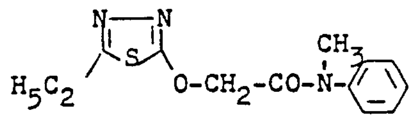 | $n_D^{22}$:1,5578 |
| 1o9 | 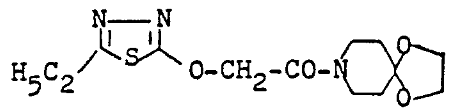 | $n_D^{22}$:1,5225 |
| 11o | 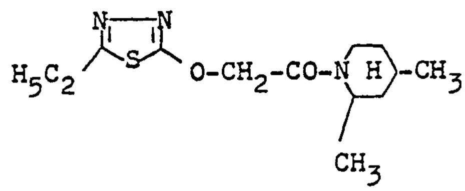 | $n_D^{22}$:1,5o19 |
| 111 | 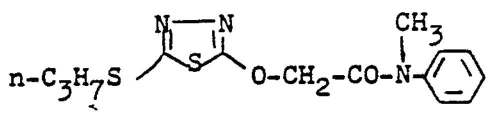 | $n_D^{23}$:1,5831 |
| 112 | 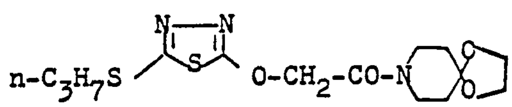 | $n_D^{22}$:1,5624 |
| 113 | 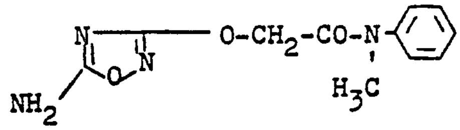 | $n_D^{22}$:1,5539 |
| 114 | 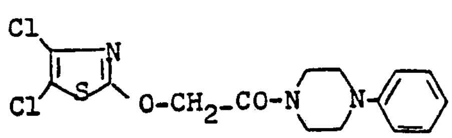 | $n_D^{22}$:1,61o2 |
| 115 | 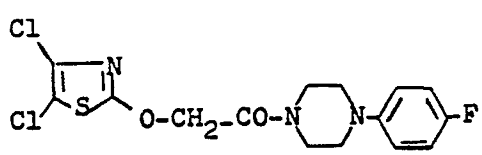 | 6o |
| 116 | 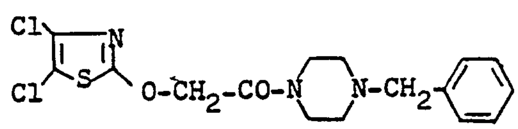 | $n_D^{22}$:1,5585 |

| Beispiel Nr. | Strukturformel | Physikal.Daten (Brechungsindex; Schmelzpunkt °C) |
|---|---|---|
| 117 | 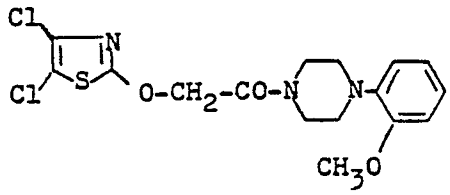 | $n_D^{22}$:1,5580 |
| 118 | 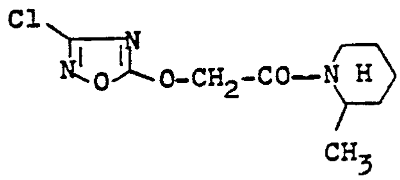 | 53 |
| 119 | 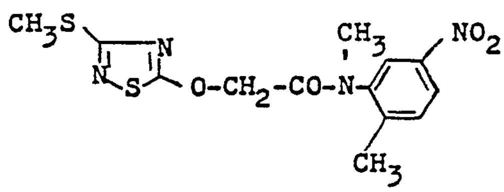 | 190 |
| 120 | 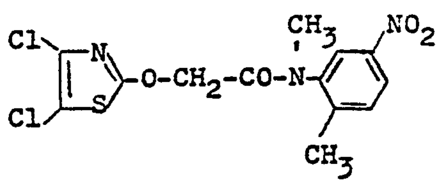 | 75 |
| 121 | 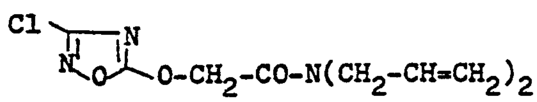 | $n_D^{20}$:1,5020 |
| 122 | 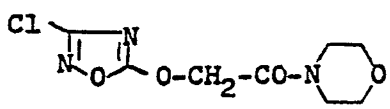 | 86 |
| 123 | 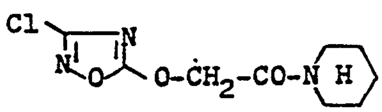 | 48 |
| 124 | 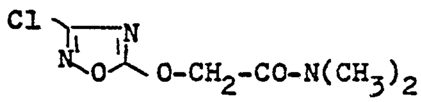 | 64 |
| 125 | 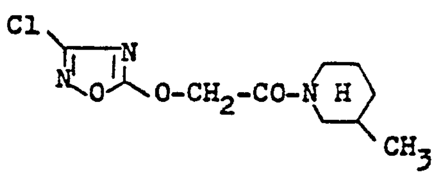 | $n_D^{20}$:1,5052 |

| Beispiel Nr. | Strukturformel | Physikal. Daten (Brechungsindex; Schmelzpunkt °C) |
|---|---|---|
| 126 | 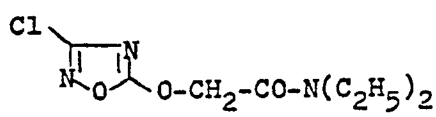 | $n_D^{20}$:1,4851 |
| 127 | 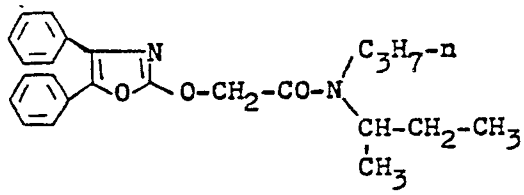 | |
| 128 | 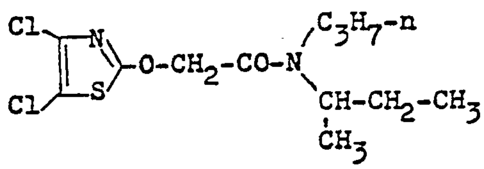 | |
| 129 | 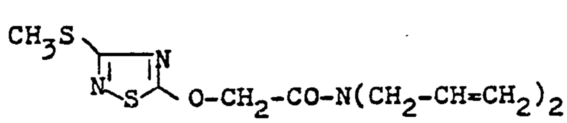 | $n_D^{21}$:1,5549 |
| 130 | 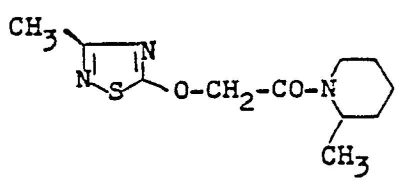 | 68 -69 |
| 131 | 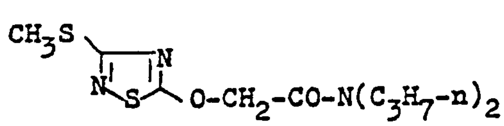 | $n_D^{21}$:1,5309 |
| 132 | 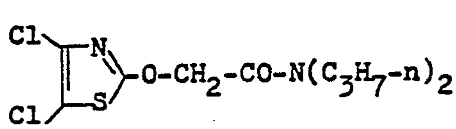 | $n_D^{22}$:1,5579 |
| 133 | 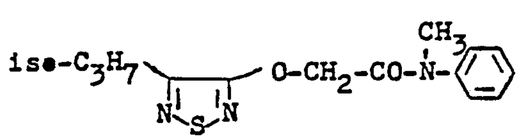 | 120 |
| 134 | 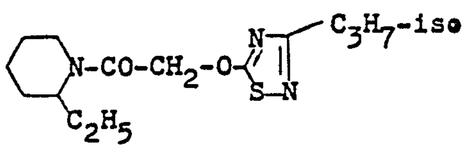 | 67 |

| Beispiel Nr. | Strukturformel | Physikal. Daten (Brechungsindex; Schmelzpunkt °C) |
|---|---|---|
| 135 | 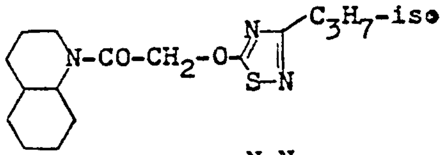 | $n_D^{23}$: 1,5291 |
| 136 | 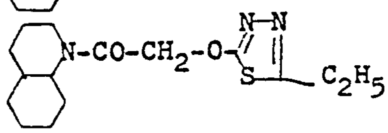 | $n_D^{23}$: 1,5414 |
| 137 | 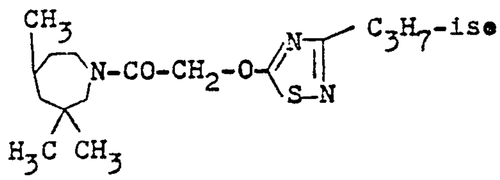 | 75 |
| 138 | 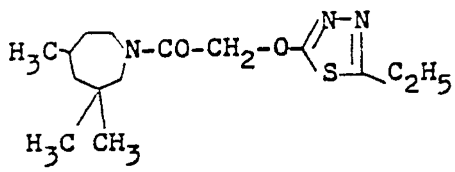 | $n_D^{23}$: 1,5212 |
| 139 | 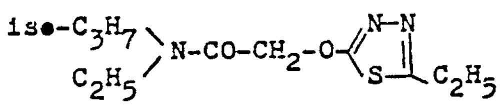 | $n_D^{23}$: 1,5125 |
| 140 | 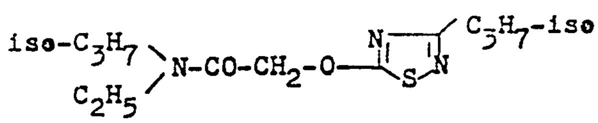 | 47 |
| 141 | 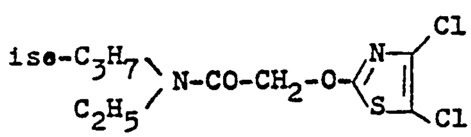 | $n_D^{23}$: 1,5385 |
| 142 | 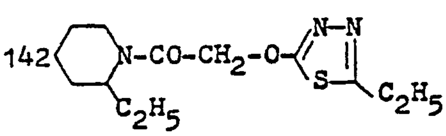 | $n_D^{23}$: 1,5268 |
| 143 | 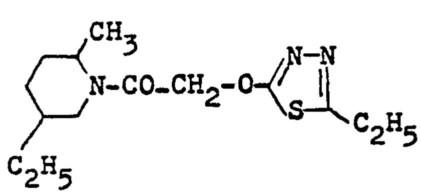 | $n_D^{23}$: 1,5172 |
| 144 | 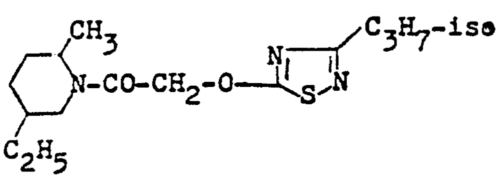 | $n_D^{23}$: 1,4980 |

| Beispiel Nr. | Strukturformel | Physikal. Daten (Brechungsindex; Schmelzpunkt °C) |
|---|---|---|
| 145 | 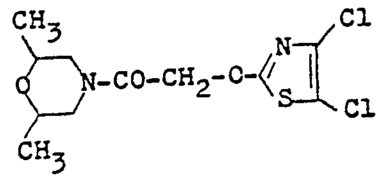 | $n_D^{23}$:1,5559 |
| 146 | 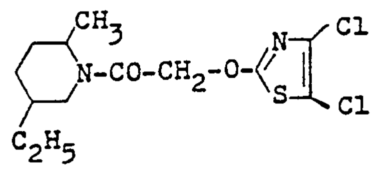 | $n_D^{23}$:1,5371 |
| 147 | 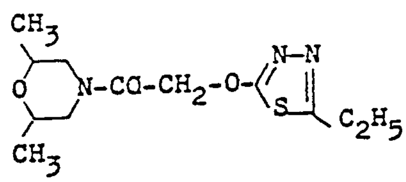 | $n_D^{23}$:1,5367 |
| 148 | 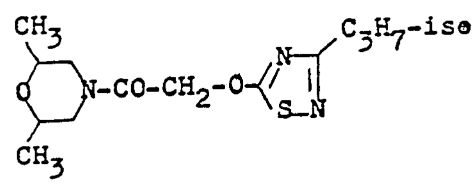 | $n_D^{23}$:1,5162 |
| 149 | 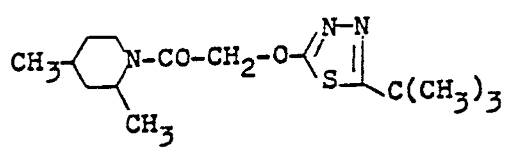 | $n_D^{23}$:1,5162 |
| 150 | 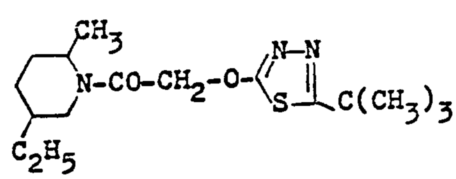 | $n_D^{23}$:1,5124 |
| 151 | 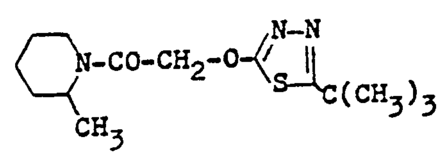 | 84-85 |
| 152 | 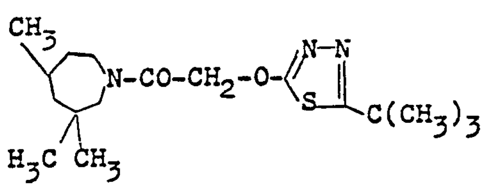 | $n_D^{23}$:1,5116 |
| 153 | 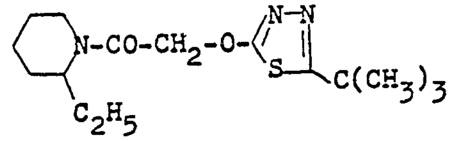 | $n_D^{23}$:1,5185 |

| Beispiel Nr. | Strukturformel | Physikal. Daten (Brechungsindex; Schmelzpunkt °C) |
|---|---|---|
| 154 | 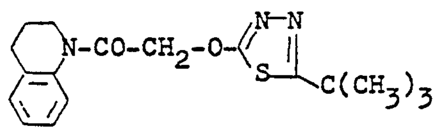 | $n_D^{23}$: 1,5713 |
| 155 | 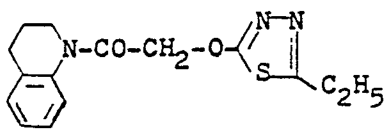 | $n_D^{23}$: 1,5865 |
| 156 | 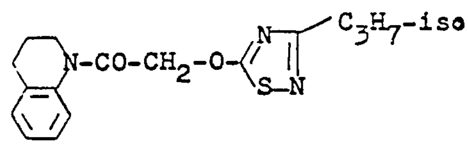 | $n_D^{23}$: 1,5760 |
| 157 | 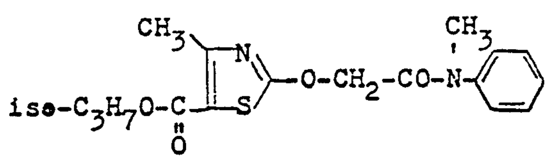 | |
| 158 | 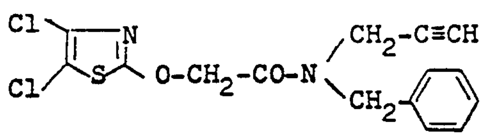 | 73 |
| 159 | 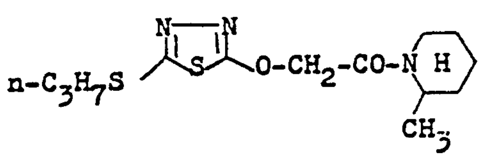 | $n_D^{20}$: 1,5461 |
| 160 | 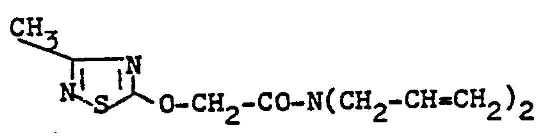 | $n_D^{20}$: 1,5193 |
| 161 | 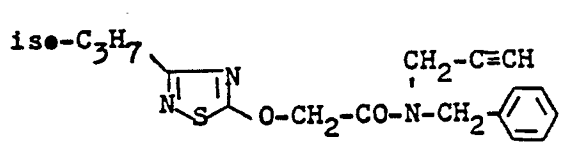 | |
| 162 | 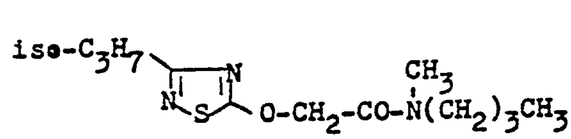 | $n_D^{20}$: 1,4947 |
| 163 | 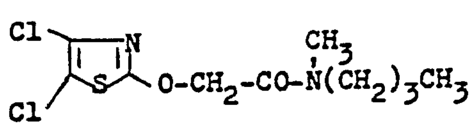 | 47 |

| Beispiel Nr. | Strukturformel | Physikal. Daten (Brechungsindex; Schmelzpunkt °C) |
|---|---|---|
| 164 | 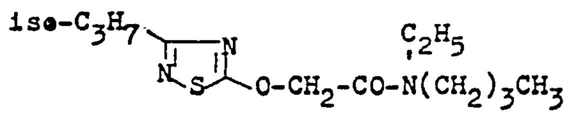 | |
| 165 | 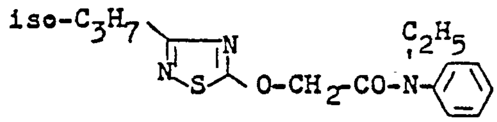 | |
| 166 | 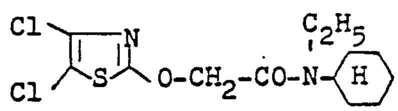 | |
| 167 | 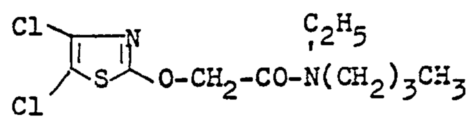 | |
| 168 | 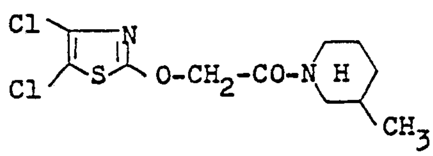 | $n_D^{20}$:1,5505 |
| 169 | 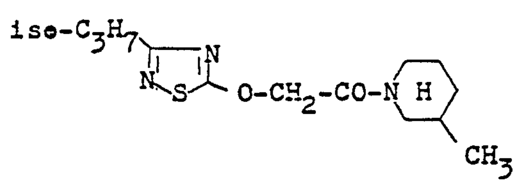 | 45 |
| 170 | 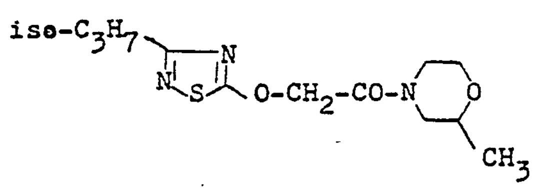 | |
| 171 | 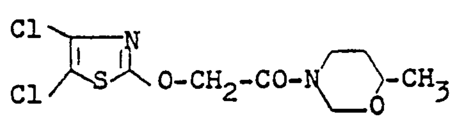 | |
| 172 | 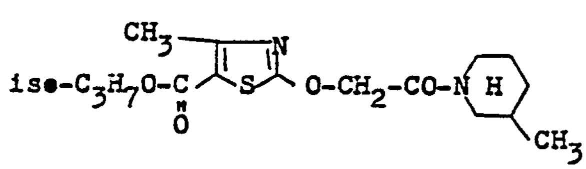 | |

| Beispiel Nr. | Strukturformel | Physikal. Daten (Brechungsindex; Schmelzpunkt °C) |
|---|---|---|
| 173 | 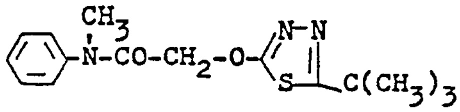 | 85-86 |
| 174 | 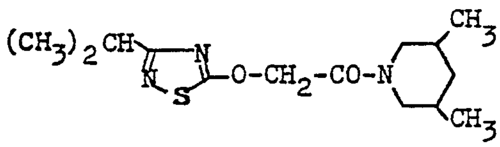 | $n_D^{21}$:1,5134 |
| 175 | 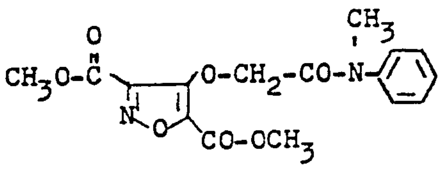 | |
| 176 | 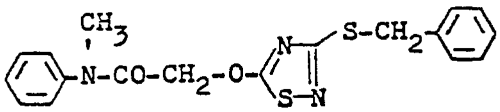 | 108 |
| 177 | 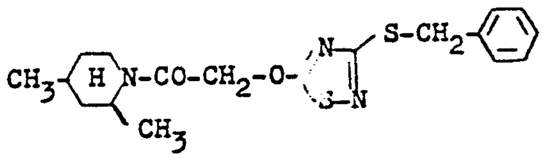 | $n_D^{19}$:1,5816 |
| 178 | 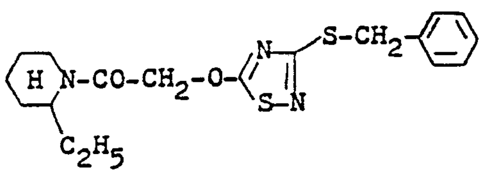 | $n_D^{19}$:1,5805 |
| 179 | 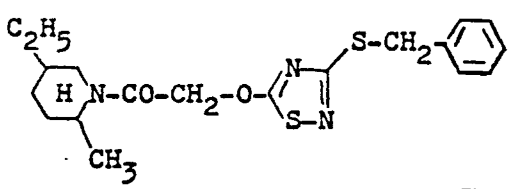 | $n_D^{19}$:1,5750 |
| 180 | 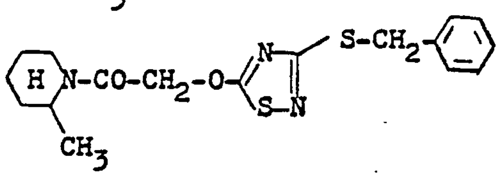 | 139 |
| 181 | 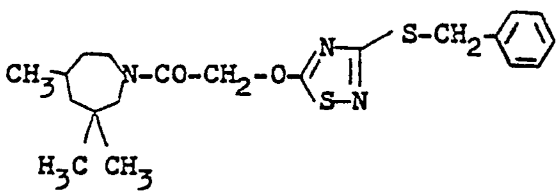 | $n_D^{19}$:1,5585 |
| 182 | 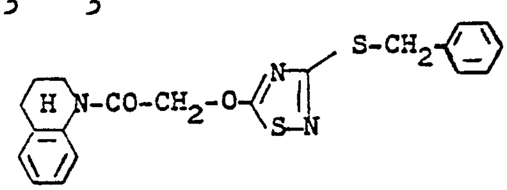 | $n_D^{19}$:1,6247 |

| Bei-spiel Nr. | Strukturformel | Physikal. Daten (Brechungs-index; Schmelz-punkt °C) |
|---|---|---|
| 183 | 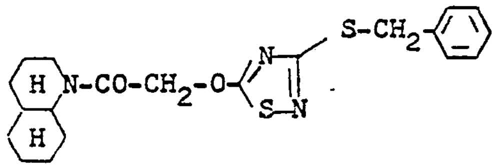 | $n_D^{19}$:1,5895 |
| 184 | 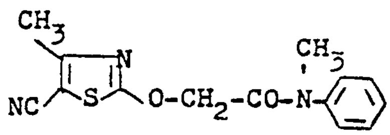 | 120 |
| 185 | 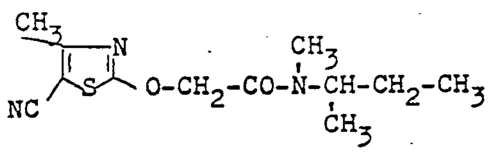 | |
| 186 | 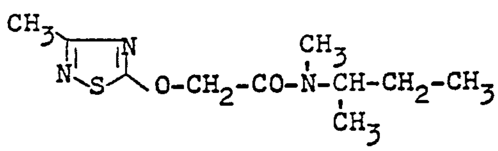 | |
| 187 | 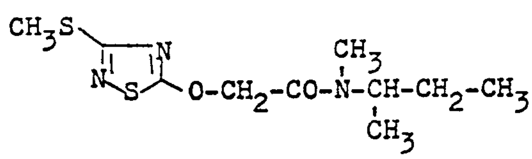 | $n_D^{20}$:1,5410 |
| 188 | 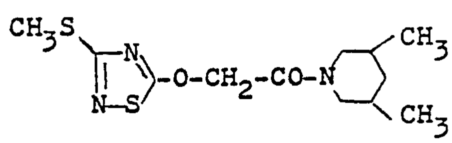 | 66 |
| 189 | 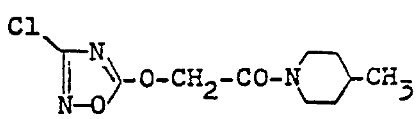 | 81 |
| 190 | 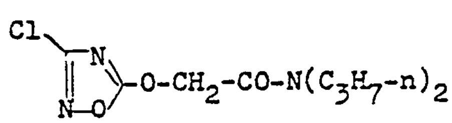 | $n_D^{20}$:1,479 |
| 191 | 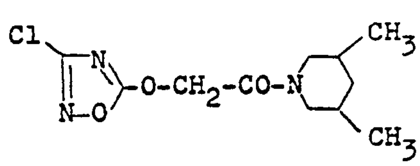 | $n_D^{20}$:1,502 |

| Beispiel Nr. | Strukturformel | Physikal. Daten (Brechungsindex; Schmelzpunkt °C) |
|---|---|---|
| 192 | 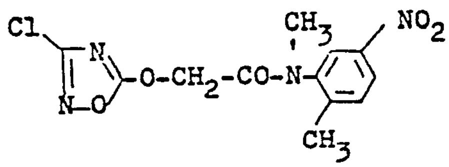 | 134 |
| 193 | 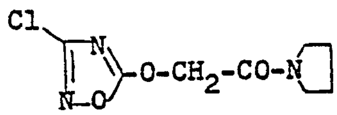 | $n_D^{20}$:1,511 |
| 194 | 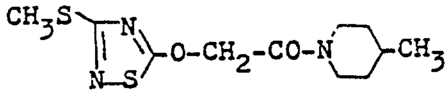 | 73 |
| 195 | 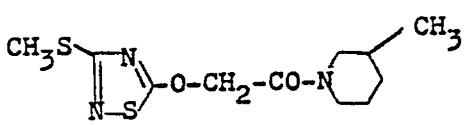 | 71 |
| 196 | 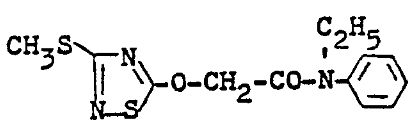 | 118 |
| 197 | 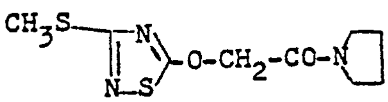 | |
| 198 | 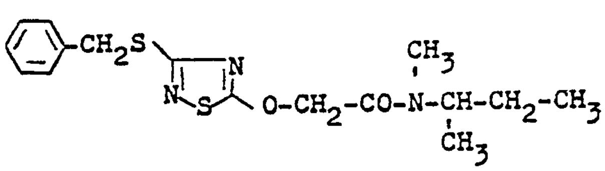 | |
| 199 | 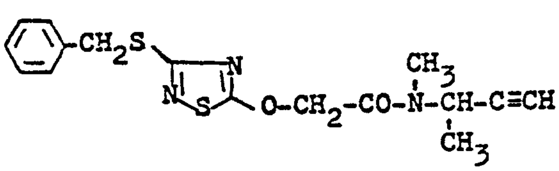 | |
| 200 | 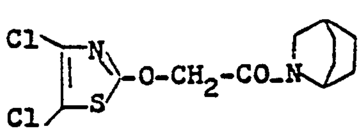 | |
| 201 | 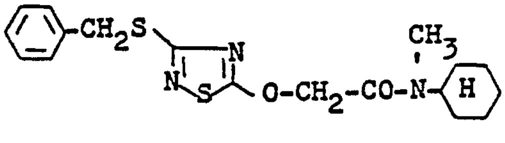 | |

| Beispiel Nr. | Strukturformel | Physikal. Daten (Brechungsindex; Schmelzpunkt °C) |
|---|---|---|
| 202 | 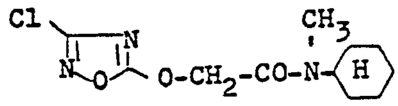 | |
| 203 | 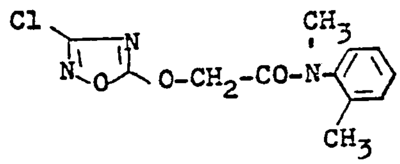 | |
| 204 | 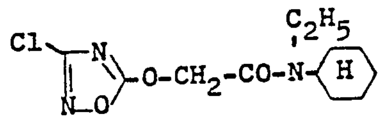 | |
| 205 | 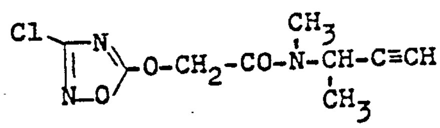 | |
| 206 | 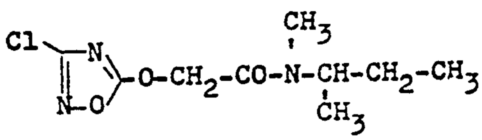 | |
| 207 | 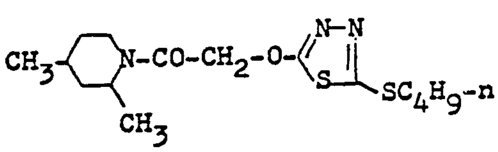 | $n_D^{23}$: 1,5550 |
| 208 | 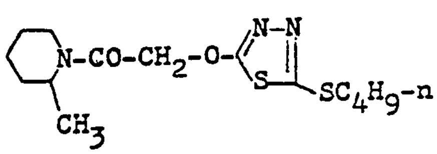 | $n_D^{23}$: 1,5738 |
| 209 | 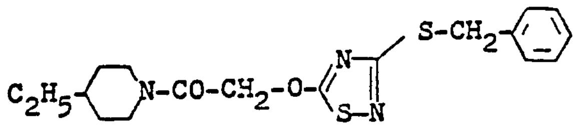 | 138 |
| 210 | 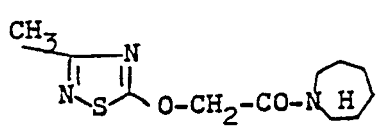 | $n_D^{20}$: 1,5130 |
| 211 | 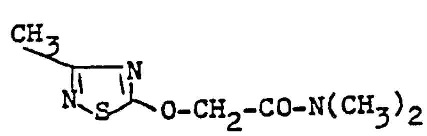 | 110 |

| Beispiel Nr. | Strukturformel | Physikal. Daten (Brechungsindex; Schmelzpunkt °C) |
|---|---|---|
| 212 | 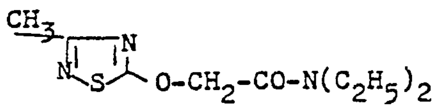 | $n_D^{20}$: 1,5130 |
| 213 | 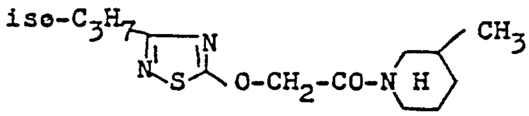 | 45 |
| 214 | 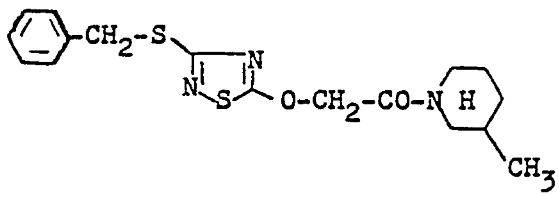 | 119 |
| 215 | 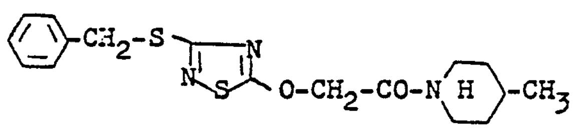 | 128 |
| 216 | 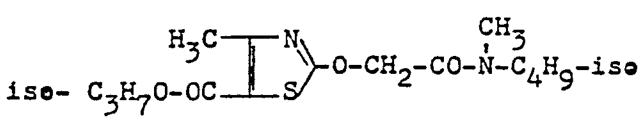 | |
| 217 | 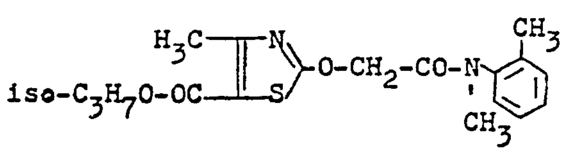 | |
| 218 | 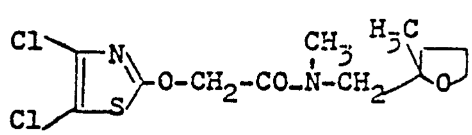 | |
| 219 | 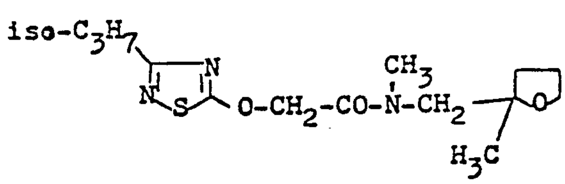 | |
| 220 | 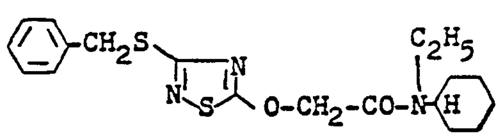 | |

| Beispiel Nr. | Strukturformel | Physikal. Daten (Brechungsindex; Schmelzpunkt °C) |
|---|---|---|
| 221 | 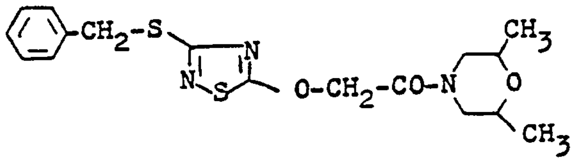 | |
| 222 | 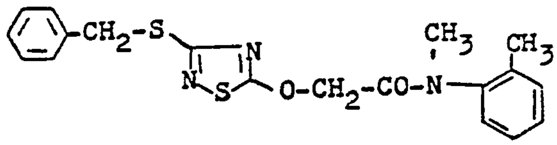 | |
| 223 | 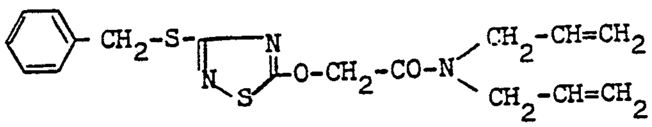 | 54 |
| 224 | 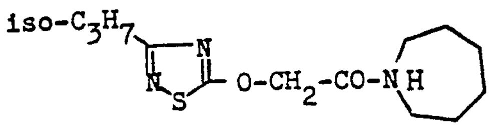 | 60 |
| 225 | 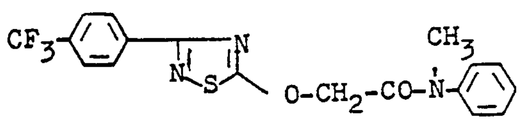 | |
| 226 | 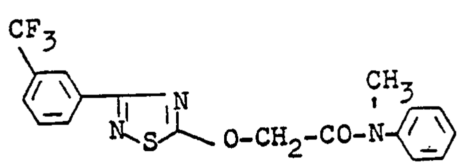 | |
| 227 | 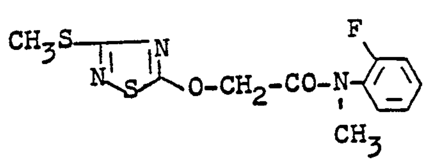 | |
| 228 | 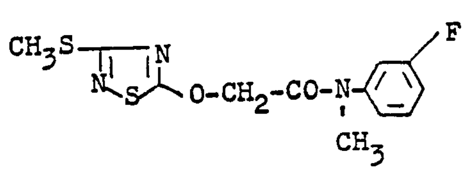 | |

| Beispiel Nr. | Strukturformel | Physikal. Daten (Brechungsindex; Schmelzpunkt °C) |
|---|---|---|
| 229 | 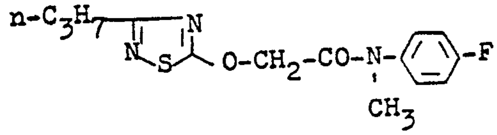 | |
| 230 | 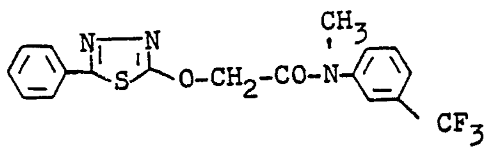 | |
| 231 | 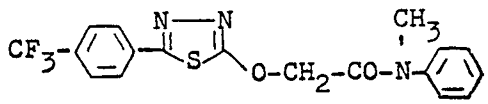 | |
| 232 | 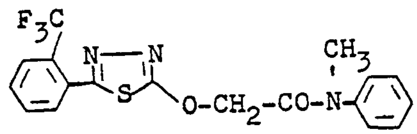 | |
| 233 | 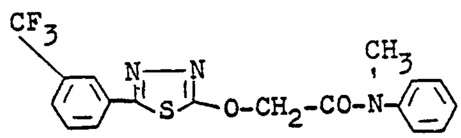 | |
| 234 | 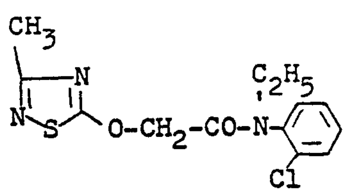 | |
| 235 | 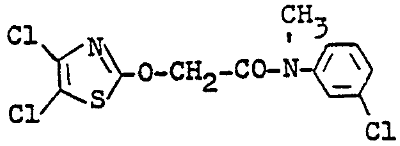 | |
| 236 | 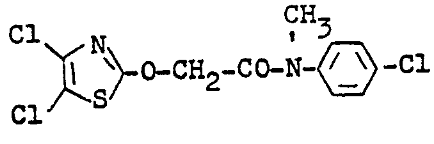 | |

| Beispiel Nr. | Strukturformel | Physikal. Daten (Brechungsindex; Schmelzpunkt °C) |
|---|---|---|
| 237 | 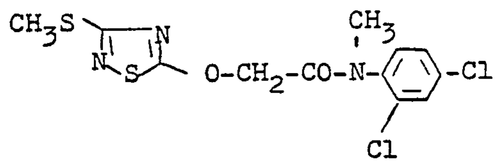 | |
| 238 | 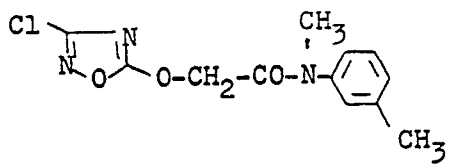 | |
| 239 | 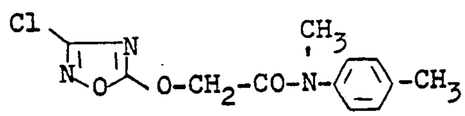 | |
| 240 | 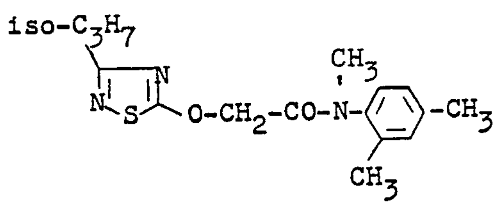 | |
| 241 | 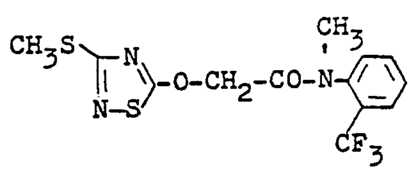 | |
| 242 | 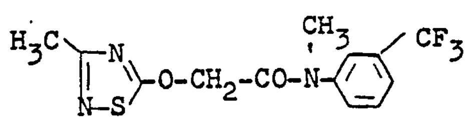 | |
| 243 | 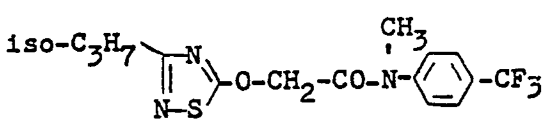 | |
| 244 | 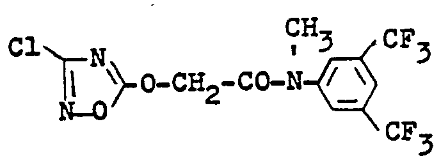 | |
| 245 | 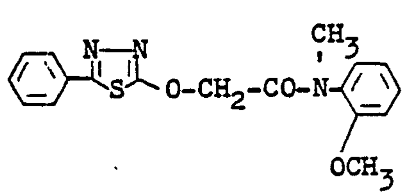 | |

| Beispiel Nr. | Strukturformel | Physikal. Daten (Brechungsindex; Schmelzpunkt °C) |
| --- | --- | --- |
| 246 | 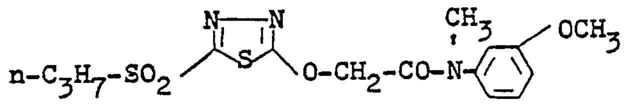 | |
| 247 | 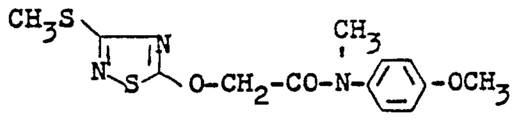 | |

Die als Ausgangsstoffe zu verwendenden Verbindungen der Formel II können wie folgt hergestellt werden:

Beispiel a

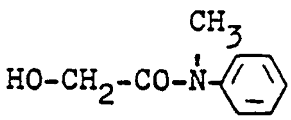

Eine Suspension aus 183,5 g (1 Mol) Chloressigsäure-N-methylanilid, 82 g (1 Mol) wasserfreiem Natriumacetat und 320 ml Toluol wird 4 Stunden auf 115-120 °C erhitzt und dann auf Raumtemperatur abgekühlt. Der Ansatz wird abgesaugt und der Rückstand mit kaltem Toluol nachgewaschen. Aus der toluolischen Lösung werden nach Abdestillieren des Lösungsmittels und Eindampfen des Rückstandes im Dampfstrahlvakuum bei einer Badtemperatur von 80-85 °C 207 g Acetoxy-essigsäure-N-methylanilid erhalten, das beim Stehen kristallisiert — GC* = 98 %ig, Schmelzpunkt 54-56 °C; Ausbeute 99 % der Theorie.

Das Reaktionsgemisch aus 211,2 g (1 Mol) Acetoxyessigsäure-N-methylanilid (98 %ig), 0,2 g Natriumhydroxid und 160 g Methanol wird 4 Stunden unter Rückfluß erhitzt. Das gemisch aus Methanol und Methylacetat wird abdestilliert. Der flüssige Destillationsrückstand -[170 g Ausbeute an Hydroxy-essigsäure-N-methylanilid, quantitativ, GC* : 98 %, Schmelzpunkt : 52-53 °C] erstarrt beim Abkühlen.

* GC = gemäß Gaschromatogramm.

Analog erhält man die nachstehenden Verbindungen der Formel II

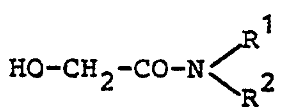

Tabelle II

| Beispiel | 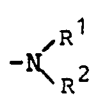 | Schmelzpunkt (°C); Brechungsindex; |
| --- | --- | --- |
| b | 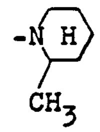 | 36 |
| c | $-N(CH_2-CH_2-OCH_3)_2$ | $n_D^{25}$:1,4662 |
| d | 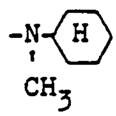 | 83 |

| Beispiel | 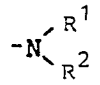 | Schmelzpunkt (°C); Brechungsindex: |
|---|---|---|
| f | 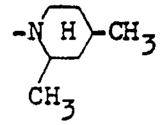 | $n_D^{23}$:1,4816 |
| g | 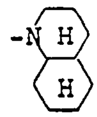 | 55 |
| h | 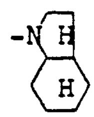 | $n_D^{23}$:1,5076 |
| i | 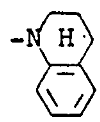 | 80 |
| j | 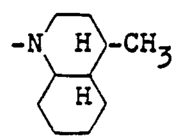 | |

Beispiel A

Pre-emergence-Test

Lösungsmittel : 5 Gewichsteile Aceton
Emulgator : 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten :

0 % = keine Wirkung (wie unbehandelte Kontrolle)
100 % = totale Vernichtung

Bei diesem Test zeigen z.B. die folgenden Verbindungen der Herstellungsbeispiele eine ausgezeichnete Wirkung :
Nr. 1, 2, 8, 17, 19, 31, 41, 99, 104, 107, 108, 118, 119, 120, 121, 122, 123, 124, 125, 126, 129, 130, 131, 132, 141, 142, 145, 154, 155, 156, 159, 160, 162, 163, 168, 180, 184, 187, 188, 189, 190, 193, 194, 195, 196

Beispiel B

Pre-emergence-Test/verpflanzter Wasserreis

Wasserbehandlung von dem Aufgehen der Samen von Reisfeldunkräutern bei unter Wasser stehenden Feldern (Topftest)
Herstellung der Werkstoffzubereitung :

Lösungsmittel : 5 Gewichtsteile Aceton
Emulgator : 1 Gewichtsteil Benzyloxypolyglycolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Testverfahren :

Wagnertöpfe (1/5 000 a) werden mit Reisfelderge gefüllt. Dann werden in jeden Topf 2 Reispflanzen (Varietät « Kinmaze ») im 2-3-Blattstadium (von etwa 10 cm Höhe) eingepflanzt.

Anschließend werden die Samen der Testpflanzen (Echinochloa crus-galli, Cyperus sp., Eleocharis acicularis und von breitblättrigen Unkräutern) in den Topf eingebracht, der ständig naß gehalten wird. Zwei Tage nach dem Einpflanzen wird jeder Topf 3 cm tief unter Wasser gesetzt und mit der Wirkstoffzubereitung begossen. Dabei hält man die Wassermenge pro Flacheneinheit zweckmäßigerweise konstant. Die Wirkstoffkonzentration in der Zubereitung spielt keine Rolle, entscheidend ist nur die Aufwandmenge des Wirkstoffs pro Flächeneinheit.

Nach der Behandlung werden bei jedem Topf zwei Tage lang jeweils 2-3 cm Wasser ablaufen gelassen, dann werden die Töpfe wieder 3 cm tief unter Wasser gesetzt. Vier Wochen nach der Behandlung mit der Wirkstoffzubereitung wird die herbizide Wirkung der erfindungsgemäßen Wirkstoffe und die Phytotoxizität gegenüber Reispflanzen gemäß der nachstehenden Skala bewertet.

Im Vergleich zur unbehandelten Kontrollfläche wird die Wirkung der erfindungsgemäßen Wirkstoffe wie folgt bewertet :

Abtötungsgrad

| | |
|---|---|
| 5 | mehr als 95 % |
| 4 | mehr als 80 % |
| 3 | mehr als 50 % |
| 2 | mehr als 30 % |
| 1 | mehr als 10 % |
| 0 | weniger als 10 % |

Im Vergleich zur unbehandelten Kontrollfläche wird die Phytotoxizität gegenüber Reispflanzen wie folgt bewertet :

Phytotoxizität

| | |
|---|---|
| 5 | mehr als 90 % (totaler Schaden) |
| 4 | mehr als 50 % |
| 3 | mehr als 30 % |
| 2 | weniger als 30 % |
| 1 | weniger als 10 % |
| 0 | 0 % (keine Phytotoxizität) |

Bei diesem Test zeigen z.B. die folgenden verbindungen der Herstellungsbeispiele eine ausgezeichnete Wirkung :

1, 6, 7, 8, 9, 10, 13, 16, 17, 19, 20, 21, 24, 26, 29, 32, 41, 51, 52, 54, 55, 56, 77, 78, 97, 99,104, 106, 108, 111, 130, 131, 132, 134, 135, 138, 141, 142, 146, 149, 153, 154, 155, 156

**Ansprüche**

1. Azolyloxyessigsäureamide der Formel I

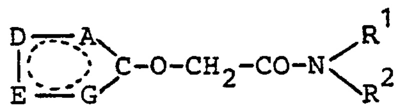

(I)

in welcher

A für $C\text{-}R^3$ oder N steht,
D für $C\text{-}R^4$ oder N steht,
E für $C\text{-}R^5$, N, O oder S steht und
G für $C\text{-}R^6$, N, O oder S steht,

mit der Maßgabe, daß wenigstens eines der Ringglieder (A, D, E oder G) für N steht und mindestens eines der Ringglieder E oder G für O oder S steht ;
in welcher weiter

$R^1$ und $R^2$, welche gleich oder verschieden sein können, einzeln für Wasserstoff, Alkyl, Cyanoalkyl, Alkoxyalkyl, Alkylthioalkyl, Alkenyl, Alkinyl, jeweils mit bis zu 10 Kohlenstoffatomen, Cycloalkyl mit bis zu 12 Kohlenstoffatomen, Aralkyl mit 1 oder 2 Kohlenstoffatomen im Alkylteil und 6 oder 10 Kohlenstoffatomen im Alkylteil und 6 oder 10 Kohlenstoffatomen im Arylteil, welcher gegebenenfalls durch Halogen substituiert ist, oder für Aryl mit 6 oder 10 Kohlenstoffatomen stehen, wobei der Arylrest durch 1 bis 3 Halogenatome, 1 bis 3 Alkyl- oder Halogenalkyl-Gruppen mit jeweils 1 bis 4 Kohlenstoffatomen, Nitro, Cyano oder Alkoxy mit 1 bis 4 Kohlenstoffatomen substituiert sein kann,
oder worin

Die Reste $R^1$ und $R^2$ zusammen mit dem Stickstoffatom, an das sie gebunden sind, einen gegebenenfalls durch 1 bis 3 Alkylgruppen mit jeweils 1 bis 5 Kohlenstoffatomen oder durch zwei geminale Alkoxygruppen mit jeweils 1 bis 3 Kohlenstoffatomen substituierten oder gegebenenfalls durch Dioxolanyliden- oder Dioxanyliden-reste spiro-cyclisch-verknüpft substituierten, gegebenenfalls teilweise ungesättigten und/oder benzannellierten Monocyclus oder Bicyclus mit bis zu 15 Kohlenstoffatomen bilden,
oder worin

Die Reste $R^1$ und $R^2$ zusammen mit dem Stickstoffatom, an das sie gebunden sind, einen gegebenenfalls durch 1 bis 3 Alkylgruppen mit jeweils 1 bis 5 Kohlenstoffatomen, durch Phenyl, welches gegebenenfalls durch $C_1$-$C_4$-Alkyl, $C_1$-$C_4$-Alkoxy, Halogen, $C_1$-$C_2$-Halogenalkyl oder Nitro substituiert ist, durch Benzyl oder Phenylethyl substituierten, gesättigten und ein weiteres Stickstoffatom, Sauerstoffatom oder Schwefelatom enthaltenden Monocyclus mit bis zu 5 Kohlenstoffatomen bilden,
in welcher ferner

$R^3$, $R^4$, $R^5$ und $R^6$, welche gleich oder verschieden sein können, einzeln für Wasserstoff, Halogen, Nitro, Cyano, Amino, $C_1$-$C_4$-Alkylamino, Di-$C_1$-$C_4$-alkylamino, $C_1$-$C_4$-Alkylcarbonylamino, $C_1$-$C_4$-Alkylcarbonyl, Carboxy, $C_1$-$C_4$-Alkoxy-carbonyl, Carbamoyl, $C_1$-$C_4$-Alkylamino-carbonyl, Di-$C_1$-$C_4$-Alkyl-amino-carbonyl, für gegebenenfalls durch Halogen, Nitro oder $C_1$-$C_4$-Alkyl substituiertes Phenyl-amino-carbonyl, für gegebenenfalls durch Halogen, Nitro, Cyano, $C_1$-$C_4$-Alkyl, $C_1$-$C_4$-Halogenalkyl oder $C_1$-$C_4$-Alkoxy substituiertes Phenyl, für gegebenenfalls durch Halogen substituiertes Benzyl oder Phenylethyl, für gegebenenfalls durch Halogen substituiertes $C_1$-$C_4$-Alkoxy, $C_2$-$C_4$-Alkenoxy, $C_2$-$C_4$-Alkinoxy, $C_1$-$C_4$-Alkoxy-carbonylmethoxy, Benzyloxy oder Phenoxy, für gegebenenfalls halogen-substituiertes $C_1$-$C_4$-Alkylthio, $C_2$-$C_4$-Alkenylthio, $C_2$-$C_4$-Alkinylthio, $C_1$-$C_4$-Alkoxy-carbonyl-methylthio, Benzylthio, Phenylthio, $C_1$-$C_4$-Alkyl-sulfinyl oder $C_1$-$C_4$-Alkylsulfonyl, für gegebenenfalls halogen-substituiertes $C_1$-$C_6$-Alkyl, $C_2$-$C_6$-Alkenyl oder $C_2$-$C_6$-Alkinyl, für Cyano-$C_1$-$C_4$-alkyl, $C_1$-$C_4$-Alkoxy-$C_1$-$C_2$-alkyl, Phenoxy- und Phenylthio-methyl, Benzyloxy- und Benzylthio-methyl, $C_1$-$C_4$-Alkylthio-$C_1$-$C_2$-alkyl, $C_1$-$C_4$-Alkyl- und Phenyl-sulfinyl-$C_1$-$C_2$-alkyl, $C_1$-$C_4$-Alkyl- und Phenyl-sulfonyl-$C_1$-$C_2$-alkyl, Carboxy-$C_1$-$C_2$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Di-$C_1$-$C_4$-alkylamino-carbonyl-$C_1$-$C_2$-alkyl, Phenylaminocarbonylalkyl oder $C_3$-$C_{12}$-Cycloalkyl stehen.

2. Azolyloxyessigsäureamid gemäß Anspruch 1 der Formel

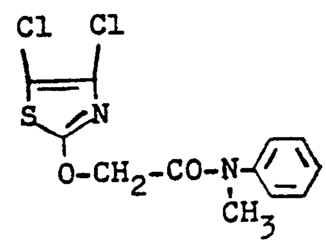

(1)

3. Azolyloxyessigsäureamid gemäß Anspruch 1 der Formel

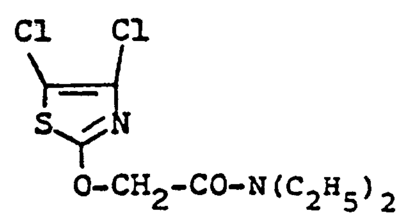

(9).

4. Azolyloxyessigsäureamid gemäß Anspruch 1 der Formel

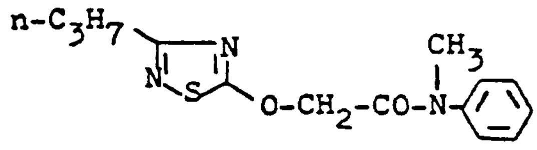

(99).

5. Azolyloxyessigsäureamid gemäß Anspruch 1 der Formel

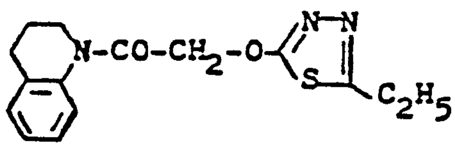

(155).

6. Verfahren zur Herstellung von Azolyloxyessigsäureamiden der Formel I

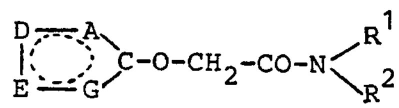

(I)

in welcher

A für C-$R^3$ oder N steht,

D für C-$R^4$ oder N steht,

E für C-$R^5$, N, O oder S steht und

G für C-$R^6$, N, O oder S steht,

mit der Maßgabe, daß wenigstens eines der Ringglieder (A, D, E oder G) für N steht und mindestens eines der Ringglieder E oder G für O oder S steht;

in welcher weiter

$R^1$ und $R^2$, welche gleich oder verschieden sein können, einzeln für Wasserstoff, Alkyl, Cyanoalkyl, Alkoxyalkyl, Alkylthioalkyl, Alkenyl, Alkinyl, jeweils mit bis zu 10 Kohlenstoffatomen, Cycloalkyl mit bis zu 12 Kohlenstoffatomen, Aralkyl mit 1 oder 2 Kohlenstoffatomen im Alkylteil und 6 oder 10 Kohlenstoffatomen im Arylteil, welcher gegebenenfalls durch Halogen substituiert ist, oder für Aryl mit 6 oder 10 kohlenstoffatomen stehen, wobei der Arylrest durch 1 bis 3 Halogenatome, 1 bis 3 Alkyl- oder Halogen-alkyl-Gruppen mit jeweils 1 bis 4 Kohlenstoffatomen, Nitro, Cyano oder Alkoxy mit 1 bis 4 Kohlenstoffatomen substituiert sein kann,

oder worin

Die Reste $R^1$ und $R^2$ zusammen mit dem Stickstoffatom, an das sie gebunden sind, einen gegebenenfalls durch 1 bis 3 Alkylgruppen mit jeweils 1 bis 5 Kohlenstoffatomen oder durch zwei geminale Alkoxygruppen mit jeweils 1 bis 3 Kohlenstoffatomen substituierten oder gegebenenfalls durch Dioxolanyliden- oder Dioxanyliden-reste spiro-cyclisch-verknüpft substituierten, gegebenenfalls teilweise ungesättiften und/oder benzannellierten Monocyclus oder Bicyclus mit bis zu 15 Kohlenstoffatomen bilden,

oder worin

Die Reste $R^1$ und $R^2$ zusammen mit dem Stickstoffatom, an das sie gebunden sind, einen gegebenenfalls durch 1 bis 3 Alkylgruppen mit jeweils 1 bis 5 Kohlenstoffatomen, durch Phenyl, welches gegebenenfalls durch $C_1$-$C_4$-Alkyl, $C_1$-$C_4$-Alkoxy, Halogen, $C_1$-$C_2$-Halogenalkyl oder Nitro substituiert ist, durch Benzyl oder Phenylethyl substituierten, gesättigten und ein weiteres Stickstoffatom, Sauerstoffatom oder Schwefelatom enthaltenen Monocyclus mit bis zu 5 Kohlenstoffatomen bilden;

in welcher ferner

$R^3$, $R^4$, $R^5$ und $R^6$, welche gleich oder verschieden sein können, einzeln für Wasserstoff, Halogen, Nitro, Cyano, Amino, $C_1$-$C_4$-Alkylamino, Di-$C_1$-$C_4$-alkylamino, $C_1$-$C_4$-Alkylcarbonylamino, $C_1$-$C_4$-Alkylcarbonyl, Carboxy, $C_1$-$C_4$-Alkoxy-carbonyl, Carbamoyl, $C_1$-$C_4$-Alkylamino-carbonyl, Di-$C_1$-$C_4$-alkyl-amino-carbonyl, für gegebenenfalls durch Halogen, Nitro oder $C_1$-$C_4$-Alkyl substituiertes Phenyl-amino-carbonyl, für gegebenenfalls durch Halogen, Nitro, Cyano, $C_1$-$C_4$-Alkyl, $C_1$-$C_4$-Halogenalkyl oder $C_1$-$C_4$-Alkoxy substituiertes Phenyl, für gegebenenfalls durch Halogen substituiertes Benzyl oder Phenylethyl, für gegebenenfalls durch Halogen substituiertes $C_1$-$C_4$-Alkoxy, $C_2$-$C_4$-Alkenoxy, $C_2$-$C_4$-Alkinocy, $C_1$-$C_4$-Alkoxy-carbonyl-methoxy, Benzyloxy oder Phenoxy, für gegebenenfalls halogen-substituiertes $C_1$-$C_4$-Alkylthio, $C_2$-$C_4$-Alkenylthio, $C_2$-$C_4$-Alkinylthio, $C_1$-$C_4$-Alkoxy-carbonyl-methylthio, Benzylthio, Phenylthio, $C_1$-$C_4$-Alkyl-sulfinyl oder $C_1$-$C_4$-Alkylsulfonyl, für gegebenenfalls halogen-substituiertes $C_1$-$C_6$-

Alkyl, $C_2$-$C_6$-Alkenyl oder $C_2$-$C_6$-Alkinyl, für Cyano-$C_1$-$C_4$-alkyl, $C_1$-$C_4$-Alkoxy-$C_1$-$C_2$-alkyl, Phenoxy- und Phenylthio-methyl, Benzyloxy- und Benzylthio-methyl, $C_1$-$C_4$-Alkylthio-$C_1$-$C_2$-alkyl, $C_1$-$C_4$-Alkyl- und Phenyl-sulfinyl-$C_1$-$C_2$-alkyl, $C_1$-$C_4$-Alkyl- und Phenyl-sulfonyl-$C_1$-$C_2$-alkyl, Carboxy-$C_1$-$C_2$-alkyl, $C_1$-$C_4$-Alkoxy-carbonyl-$C_1$-$C_2$-alkyl, $C_1$-$C_4$-Alkyl-amino-carbonyl-$C_1$-$C_2$-alkyl, Di-$C_1$-$C_4$-alkylamino-carbonyl-$C_1$-$C_2$-alkyl, Phenylaminocarbonylalkyl oder $C_3$-$C_{12}$-Cycloalkyl stehen,
dadurch gekennzeichnet, daß man Hydroxyessigsäureamide der Formel II

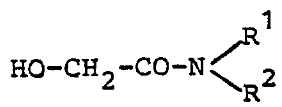

(II)

in welcher

$R^1$ und $R^2$ die bei Formel I angegebene Bedeutung haben, mit Halogenazolen der Formel III

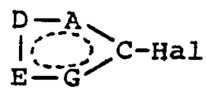

(III)

worin

A, D, E und G die bei Formel I angegebene Bedeutung haben und
Hal für Chlor, Brom oder Jod steht,

gegebenenfalls in Gegenwart eines Säureakzeptors und gegebenenfalls unter Verwendung eines Verdünnungsmittels umsetzt.

7. Herbizide Mittel, gekennzeichnet durch einen Gehalt an mindestens einem Azolyloxyessigsäureamid der Formel I gemäß Anspruch 1 bzw. Anspruch 6.

8. Verfahren zur Bekämpfung von unerwünschtem Pflanzenwachstum, dadurch gekennzeichnet, daß man mindestens ein Azolyloxyessigsäureamid der Formel I gemäß Anspruch 1 bzw. Anspruch 6 auf die unerwünschten Pflanzen oder ihren Lebensraum einwirken läßt.

9. Verwendung von Azolyloxyessigsäureamiden der Formel I gemäß Anspruch 1 bzw. Anspruch 6 zur Bekämpfung von unerwünschtem Pflanzenwachstum.

10. Verfahren zur Herstellung von herbiziden Mitteln, dadurch gekennzeichnet, daß man Azolyloxyessigsäureamide der Formel I gemäß Anspruch 1 bzw. Anspruch 6 mit Streckmitteln und/oder oberflächenaktiven Mitteln vermischt.

**Claims**

1. Azolyloxy-acetic acid amides of the formula I

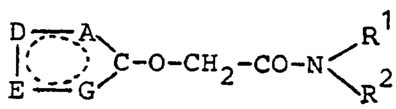

(I)

in which

A represents C-$R^3$ or N,
D represents C-$R^4$ or N,
E represents C-$R^5$, N, O or S and
G represents C-$R^6$, N, O or S,

with the proviso that at least one of the ring members (A, D, E or G) represents N and at least one of the ring members E or G represents O or S ;
in which, furthermore,

$R^1$ and $R^2$, which can be identical or different, individually represent hydrogen, alkyl, cyanoalkyl, alkoxyalkyl, alkylthioalkyl, alkenyl, alkinyl, in each case with up to 10 carbon atoms, cycloalkyl with up to 12 carbon atoms, aralkyl which has 1 or 2 carbon atoms in the alkyl part and 6 or 10 carbon atoms in the aryl part and which is optionally substituted by halogen, or aryl with 6 or 10 carbon atoms, it being possible for the aryl radical to be substituted by 1 to 3 halogen atoms, 1 to 3 alkyl or halogenoalkyl groups with in each case 1 to 4 carbon atoms, nitro, cyano or alkoxy with 1 to 4 carbon atoms,
or wherein

the radicals $R^1$ and $R^2$, together with the nitrogen atom to which they are bonded, form an optionally partially unsaturated and/or benzo-fused monocyclic or bicyclic radical which has up to 15 carbon atoms

and is optionally substituted by 1 to 3 alkyl groups with in each case 1 to 5 carbon atoms or by two geminal alkoxy groups with in each case 1 to 3 carbon atoms or is optionally substituted by dioxolanylidene or dioxanylidene radicals linked in a spiro-cyclic manner,

or wherein

the radicals $R^1$ and $R^2$, together with the nitrogen atom to which they are bonded, form a saturated monocyclic radical which has up to 5 carbon atoms, contains a further nitrogen atom, oxygen atom or sulphur atom and is optionally substituted by 1 to 3 alkyl groups with in each case 1 to 5 carbon atoms, by phenyl which is optionally substituted by $C_1$-$C_4$-alkyl, $C_1$-$C_4$-alkoxy, halogen, $C_1$-$C_2$-halogenoalkyl or nitro, or by benzyl or phenylethyl ;

and in which, furthermore,

$R^3$, $R^4$, $R^5$ and $R^6$, which can be identical or different, individually represent hydrogen, halogen, nitro, cyano, amino, $C_1$-$C_4$-alkylamino, di-$C_1$-$C_4$-alkylamino, $C_1$-$C_4$-alkylcarbonylamino, $C_1$-$C_4$-alkylcarbonyl, carboxyl, $C_1$-$C_4$-alkoxy-carbonyl, carbamoyl, $C_1$-$C_4$-alkylamino-carbonyl, di-$C_1$-$C_4$-alkylamino-carbonyl, phenyl-amino-carbonyl which is optionally substituted by halogen, nitro or $C_1$-$C_4$-alkyl, phenyl which is optionally substituted by halogen, nitro, cyano, $C_1$-$C_4$-alkyl, $C_1$-$C_4$-halogenoalkyl or $C_2$-$C_4$-alkoxy, optionally halogen-substituted benzyl or phenylethyl, optionally halogen-substituted $C_1$-$C_4$-alkoxy, $C_2$-$C_4$-alkenoxy, $C_2$-$C_4$-alkinoxy, $C_1$-$C_4$-alkoxy-carbonylmethoxy, benzyloxy or phenoxy, optionally halogen-substituted $C_1$-$C_4$-alkylthio, $C_2$-$C_4$-alkenylthio, $C_2$-$C_4$-alkinylthio, $C_1$-$C_4$-alkoxy-carbonylmethylthio, benzylthio, phenylthio, $C_1$-$C_4$-alkyl-sulphinyl or $C_1$-$C_4$-alkylsulphonyl, optionally halogen-substituted $C_1$-$C_6$-alkyl, $C_2$-$C_6$-alkenyl or $C_2$-$C_6$-alkinyl, cyano-$C_1$-$C_4$-alkyl, $C_1$-$C_4$-alkoxy-$C_1$-$C_2$-alkyl, phenoxy- or phenylthio-methyl, benzyloxy- or benzylthio-methyl, $C_1$-$C_4$-alkylthio-$C_1$-$C_2$-alkyl, $C_1$-$C_4$-alkyl- or phenyl-sulphinyl-$C_1$-$C_2$-alkyl, $C_1$-$C_4$-alkyl- or phenyl-sulphonyl-$C_1$-$C_2$-alkyl, carboxy-$C_1$-$C_2$-alkyl, $C_1$-$C_4$-alkoxy-carbonyl-$C_1$-$C_2$-alkyl, $C_1$-$C_4$-alkylamino-carbonyl-$C_1$-$C_2$-alkyl, di-$C_1$-$C_4$-alkylamino-carbonyl-$C_1$-$C_2$-alkyl, phenylaminocarbonylalkyl or $C_3$-$C_{12}$-cycloalkyl.

2. Azolyloxy-acetic acid amide according to Claim 1 of the formula

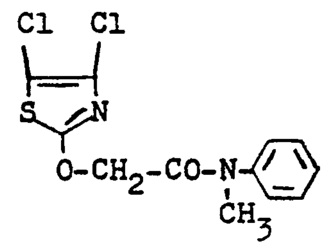

(1)

3. Azolyloxy-acetic acid amide according to Claim 1 of the formula

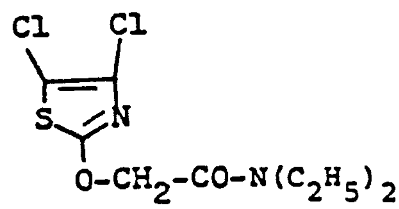

(9)

4. Azolyloxy-acetic acid amide according to Claim 1 of the formula

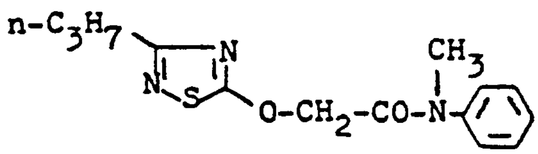

(99)

5. Azolyloxy-acetic acid amide according to Claim 1 of the formula

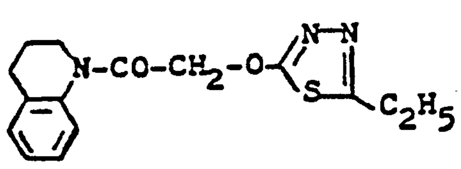

(155)

6. Process for the preparation of azolyloxy-acetic acid amides of the formula I

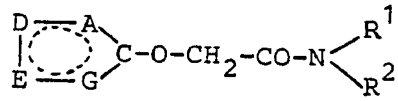

(I)

in which

A represents C-$R^3$ or N,

D represents C-$R^4$ or N,

E represents C-$R^5$, N, O or S and

G represents C-$R^6$, N, O or S,

with the proviso that at least one of the ring members (A. D, E or G) represents N and at least one of the ring members E or G represents O or S ;

in which, furthermore,

$R^1$ and $R^2$, which can be identical or different, individually represent hydrogen, alkyl, cyanoalkyl, alkoxyalkyl, alkylthioalkyl, alkenyl, alkinyl, in each case with up to 10 carbon atoms, cycloalkyl with up to 12 carbon atoms, aralkyl which has 1 or 2 carbon atoms in the alkyl part and 6 or 10 carbon atoms in the aryl part and which is optionally substituted by halogen, or aryl with 6 or 10 carbon atoms, it being possible for the aryl radical to be substituted by 1 to 3 halogen atoms, 1 to 3 alkyl or halogenoalkyl groups with in each case 1 to 4 carbon atoms,. nitro, cyano or alkoxy with 1 to 4 carbon atoms,

or wherein

the radicals $R^1$ and $R^2$, together with the nitrogen atom to which they are bonded, form an optionally partially unsaturated and/or benzo-fused monocyclic or bicyclic radical which has up to 15 carbon atoms and is optionally substituted by 1 to 3 alkyl groups with in each case 1 to 5 carbon atoms or by two geminal alkoxy groups with in each case 1 to 3 carbon atoms or is optionally substituted by dioxolanylidene or dioxanylidene radicals linked in a spiro-cyclic manner,

or wherein

the radicals $R^1$ and $R^2$, together with the nitrogen atom to which they are bonded, form a saturated monocyclic radical which has up to 5 carbon atoms, contains a further nitrogen atom, oxygen atom or sulphur atom and is optionally substituted by 1 to 3 alkyl groups with in each case 1 to 5 carbon atoms, by phenyl which is optionally substituted by $C_1$-$C_4$-alkyl, $C_1$-$C_4$-alkoxy, halogen, $C_1$-$C_2$-halogenoalkyl or nitro, or by benzyl or phenylethyl ;

and in which, furthermore,

$R^3$, $R^4$, $R^5$ and $R^6$, which can be identical or different, individually represent hydrogen, halogen, nitro, cyano, amino, $C_1$-$C_4$-alkylamino, di-$C_1$-$C_4$-alkylamino, $C_1$-$C_4$-alkylcarbonylamino, $C_1$-$C_4$-alkylcarbonyl, carboxyl, $C_1$-$C_4$-alkoxy-carbonyl, carbamoyl, $C_1$-$C_4$-alkylamino-carbonyl, di-$C_1$-$C_4$-alkylamino-carbonyl, phenyl-amino-carbonyl which is optionally substituted by halogen, nitro or $C_1$-$C_4$-alkyl, phenyl which is optionally substituted by halogen, nitro, cyano $C_1$-$C_4$-alkyl, $C_1$-$C_4$-halogeno-alkyl or $C_1$-$C_4$-alkoxy, optionally halogen-substituted benzyl or phenylethyl, optionally halogen-substituted $C_1$-$C_4$-alkoxy, $C_2$-$C_4$-alkenoxy, $C_2$-$C_4$-alkinoxy, $C_1$-$C_4$-alkoxy-carbonylmethoxy, benzyloxy or phenoxy, optionally halogen substituted $C_1$-$C_4$-alkylthio, $C_2$-$C_4$-alkenylthio, $C_2$-$C_4$-alkinylthio, $C_1$-$C_4$-alkoxy-carbonylmethylthio, benzylthio, phenylthio, $C_1$-$C_4$-alkyl-sulphinyl or $C_1$-$C_4$-alkylsulphonyl, optionally halogen-substituted $C_1$-$C_6$-alkyl, $C_2$-$C_6$-alkenyl or $C_2$-$C_6$-alkinyl, cyano-$C_1$-$C_4$-alkyl, $C_1$-$C_4$-alkoxy-$C_1$-$C_2$-alkyl, phenoxy- or phenylthiomethyl, benzyloxy- or benzylthio-methyl, $C_1$-$C_4$-alkylthio-$C_1$-$C_2$-alkyl, $C_1$-$C_4$-alkyl- or phenyl-sulphinyl-$C_1$-$C_2$-alkyl, $C_1$-$C_4$-alkyl- or phenyl-sulphonyl-$C_1$-$C_2$-alkyl, carboxy-$C_1$-$C_2$-alkyl, $C_1$-$C_4$-alkoxy-carbonyl-$C_1$-$C_2$-alkyl, $C_1$-$C_4$-alkylamino-carbonyl-$C_1$-$C_2$-alkyl, di-$C_1$-$C_4$-alkylamino-carbonyl-$C_1$-$C_2$-alkyl, phenylaminocarbonylalkyl or $C_3$-$C_{12}$-cycloalkyl,

characterised in that hydroxy-acetic acid amides of the formula II

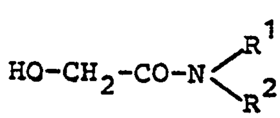

(II)

in which

$R^1$ and $R^2$ have the meaning given under formula I, are reacted with halogenoazoles of the formula III

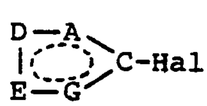

(III)

wherein

A, D, E and G have the meaning given under formula I and

Hal represents chlorine, bromine or iodine,

if appropriate in the presence of an acid acceptor and if appropriate using a diluent.

7. Herbicidal agents, characterised in that they contain at least one azolyloxy-acetic acid amide of the formula I according to Claim 1 and Claim 6.

8. Process for combating undesired plant growth, characterised in that at least one azolyloxy-acetic acid amide of the formula I according to Claim 1 and Claim 6 is allowed to act on the undesired plants or their environment.

9. Use of azolyloxy-acetic acid amides of the formula I according to Claim 1 and Claim 6 for combating undesired plant growth.

10. Process for the preparation of herbicidal agents, characterised in that azolyloxy-acetic acid amides of the formula I according to Claim 1 and Claim 6 are mixed with extenders and/or surface-active agents.

**Revendications**

1. Amides d'acides azolyloxyacétiques de formule I :

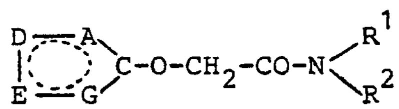

(I)

dans laquelle

A représente C-$R^3$ ou N,

D représente C-$R^4$ ou N,

E représente C-$R^5$, N, O ou S, et

G représente C-$R^6$, N, O ou S,

à condition qu'au moins un des termes cycliques (A, D, E ou G) représente N et qu'au moins un des termes cycliques E ou G représente O ou S ;

formule dans laquelle également :

$R^1$ et $R^2$, qui peuvent être identiques ou différents, représentent chacun un atome d'hydrogène, un groupe alkyle, un groupe cyanoalkyle, un groupe alcoxyalkyle, un groupe alkylthioalkyle, un groupe alcényle, un groupe alcinyle, chacun de ces groupes contenant jusqu'à 10 atomes de carbone, un groupe cycloalkyle contenant jusqu'à 12 atomes de carbone, un groupe aralkyle contenant 1 ou 2 atomes de carbone dans la fraction alkyle et 6 ou 10 atomes de carbone dans la fraction aryle, qui est éventuellement substituée par un atome d'halogène, ou encore un groupe aryle contenant 6 ou 10 atomes de carbone, ce groupe aryle pouvant être substitué par un à trois atomes d'halogènes, par un à trois groupes alkyle ou halogénalkyle contenant chacun 1 à 4 atomes de carbone, par un groupe nitro, par un groupe cyano ou par un groupe alcoxy contenant 1 à 4 atomes de carbone,

ou

les radicaux $R^1$ et $R^2$, ensemble avec l'atome d'azote auquel ils sont reliés, forment un noyau monocyclique ou bicyclique contenant jusqu'à 15 atomes de carbone, éventuellement partiellement insaturé et/ou condensé en noyaux benzène, éventuellement substitué par 1 à 3 groupes alkyle contenant chacun 1 à 5 atomes de carbone ou par deux groupes alcoxy géminés contenant chacun 1 à 3 atomes de carbone, ou encore éventuellement substitué, avec une liaison spiro-cyclique, par des groupes dioxolanylidène ou dioxanylidène,

ou encore

les radicaux $R^1$ et $R^2$, ensemble avec l'atome d'azote auquel ils sont reliés, forment un noyau monocyclique saturé contenant jusqu'à 5 atomes de carbone et un atome supplémentaire d'azote, d'oxygène ou de soufre et éventuellement substitué par un à trois groupes alkyle contenant chacun 1 à 5 atomes de carbone, par un groupe phényle (éventuellement substitué par un groupe alkyle en $C_1$-$C_4$, par un groupe alcoxy en $C_1$-$C_4$, par un atome d'halogène, par un groupe halogénalkyle en $C_1$-$C_2$ ou par un groupe nitro), par un groupe benzyle ou par un groupe phényléthyle,

formule dans laquelle également :

$R^3$, $R^5$ et $R^6$, qui peuvent être identiques ou différents, représentent chacun un atome d'hydrogène, un atome d'halogène, un groupe nitro, un groupe cyano, un groupe amino, un groupe alkyl (en $C_1$-$C_4$)-amino, un groupe di-alkyl (en $C_1$-$C_4$) amino, un groupe alkyl (en $C_1$-$C_4$) carbonylamino, un groupe alkyl (en $C_1$-$C_4$) carbonyle, un groupe carboxy, un groupe alcoxy (en $C_1$-$C_4$) carbonyle, un groupe carbamoyle, un groupe alkyl (en $C_1$-$C_4$) aminocarbonyle, un groupe di-alkyl (en $C_1$-$C_4$) aminocarbonyle, un groupe phénylaminocarbonyle éventuellement substitué par un atome d'halogène, par un groupe nitro ou par un

groupe alkyle en $C_1$-$C_4$, un groupe phényle éventuellement substitué par un atome d'halogène, par un groupe nitro, par un groupe cyano, par un groupe alkyle en $C_1$-$C_4$, par un groupe halogénalkyle en $C_1$-$C_4$ ou par un groupe alcoxy en $C_1$-$C_4$, un groupe benzyle ou un groupe phényléthyle éventuellement substitué par un atome d'halogène, un groupe alcoxy en $C_1$-$C_4$, un groupe alcénoxy en $C_2$-$C_4$, un groupe alcinoxy en $C_2$-$C_4$, un groupe alcoxy (en $C_1$-$C_4$) carbonylméthoxy, un groupe benzyloxy ou un groupe phénoxy éventuellement substitué par un atome d'halogène, un groupe alkyl (en $C_1$-$C_4$) thio, un groupe alcényl (en $C_2$-$C_4$) thio, un groupe alcinyl (en $C_2$-$C_4$) thio, un groupe alcoxy (en $C_1$-$C_4$)-carbonylméthylthio, un groupe benzylthio, un groupe phénylthio, un groupe alkyl (en $C_1$-$C_4$) sulfinyle, ou un groupe alkyl (en $C_1$-$C_4$) sulfonyle éventuellement substitué par un atome d'halogène, un groupe alkyle en $C_1$-$C_6$, un groupe alcényle en $C_2$-$C_6$ ou un groupe alcinyle en $C_2$-$C_6$ éventuellement substitué par un atome d'halogène, un groupe cyanoalkyle en $C_1$-$C_4$, un groupe alcoxy (en $C_1$-$C_4$) alkyle en $C_1$-$C_2$, un groupe phénoxy- et phénylthiométhyle, un groupe benzyloxy- ou benzylthiométhyle, un groupe alkyl (en $C_1$-$C_4$) thioalkyle en $C_1$-$C_2$, un groupe alkyl (en $C_1$-$C_4$)- ou phénylsulfinylalkyle en $C_1$-$C_2$, un groupe alkyl (en $C_1$-$C_4$)- ou phénylsulfonylalkyle en $C_1$-$C_2$, un groupe carboxyalkyle en $C_1$-$C_2$, un groupe alcoxy (en $C_1$-$C_4$) carbonylalkyle en $C_1$-$C_2$, un groupe alkyl (en $C_1$-$C_4$) aminocarbonylalkyle en $C_1$-$C_2$, un groupe dialkyl (en $C_1$-$C_4$) aminocarbonylalkyle en $C_1$-$C_2$, un groupe phénylaminocarbonylalkyle ou un groupe cycloalkyle en $C_3$-$C_{12}$.

2. Amide d'acide azolyloxyacétique suivant la revendication 1 et répondant à la formule :

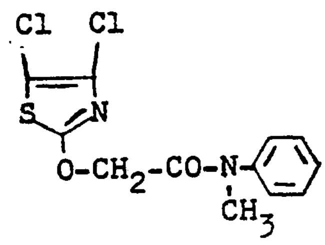

(1)

3. Amide d'acide azolyloxyacétique suivant la revendication 1 et répondant à la formule :

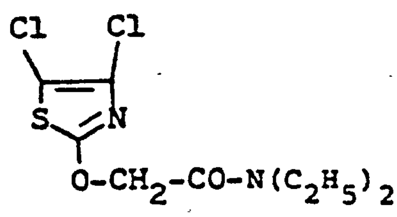

(9)

4. Amide d'acide azolyloxyacétique suivant la revendication 1 et répondant à la formule :

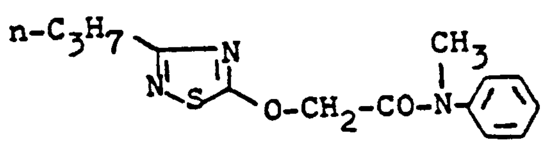

(99)

5. Amide d'acide azolyloxyacétique suivant la revendication 1 et répondant à la formule :

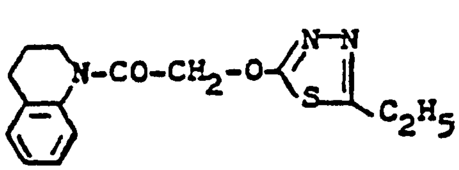

(155)

6. Procédé de préparation d'amides d'acides azolyloxyacétiques de formule I :

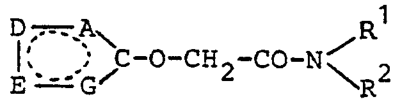

(I)

dans laquelle

A représente C-$R^3$ ou N,

D représente C-$R^4$ ou N,

E représente C-$R^5$, N, O ou S, et

G représente C-$R^6$, N, O ou S,

à condition qu'au moins un des termes cycliques (A, D, E ou G) représente N et qu'au moins un des termes cycliques E ou G représente O ou S ;

formule dans laquelle également :

$R^1$ et $R^2$, qui peuvent être identiques ou différents, représentent chacun un atome d'hydrogène, un groupe alkyle, un groupe cyanoalkyle, un groupe alcoxyalkyle, un groupe alkylthioalkyle, un groupe alcényle, un groupe alcinyle, chacun de ces groupes contenant jusqu'à 10 atomes de carbone, un groupe cycloalkyle contenant jusqu'à 12 atomes de carbone, un groupe aralkyle contenant 1 ou 2 atomes de carbone dans la fraction alkyle et 6 ou 10 atomes de carbone dans la fraction aryle, qui est éventuellement substituée par un atome d'halogène, ou encore un groupe aryle contenant 6 ou 10 atomes de carbone, le groupe aryle pouvant être substitué par un à trois atomes d'halogènes, par un à trois groupes alkyle ou halogénalkyle contenant chacun 1 à 4 atomes de carbone, par un groupe nitro, par un groupe cyano ou par un groupe alcoxy contenant 1 à 4 atomes de carbone,

ou

les radicaux $R^1$ et $R^2$, ensemble avec l'atome d'azote auquel ils sont reliés, forment un noyau monocyclique ou bicyclique contenant jusqu'à 15 atomes de carbone, éventuellement partiellement insaturé et/ou condensé en noyaux benzène, éventuellement substitué par 1 à 3 groupes alkyle contenant chacun 1 à 5 atomes de carbone ou par deux groupes alcoxy géminés contenant chacun 1 à 3 atomes de carbone, ou encore éventuellement substitué, avec une liaison spiro-cyclique, par des groupes dioxolanylidène ou dioxanylidène,

ou encore

les radicaux $R^1$ et $R^2$, ensemble avec l'atome d'azote auquel ils sont reliés, forment un noyau monocyclique saturé contenant jusqu'à 5 atomes de carbone et un atome supplémentaire d'azote, d'oxygène ou de soufre et éventuellement substitué par un à trois groupes alkyle contenant chacun 1 à 5 atomes de carbone, par un groupe phényle (éventuellement substitué par un groupe alkyle en $C_1$-$C_4$, par un groupe alcoxy en $C_1$-$C_4$, par un atome d'halogène, par un groupe halogénalkyle en $C_1$-$C_2$ ou par un groupe nitro), par un groupe benzyle ou par un groupe phényléthyle,

formule dans laquelle également :

$R^3$, $R^4$, $R^5$ et $R^6$, qui peuvent être identiques ou différents, représentent chacun un atome d'hydrogène, un atome d'halogène, un groupe nitro, un groupe cyano, un groupe amino, un groupe alkyl (en $C_1$-$C_4$)-amino, un groupe di-alkyl (en $C_1$-$C_4$) amino, un groupe alkyl (en $C_1$-$C_4$) carbonylamino, un groupe alkyl (en $C_1$-$C_4$) carbonyle, un groupe carboxy, un groupe alcoxy (en $C_1$-$C_4$) carbonyle, un groupe carbamoyle, un groupe alkyl (en $C_1$-$C_4$) aminocarbonyle, un groupe di-alkyl (en $C_1$-$C_4$) aminocarbonyle, un groupe phénylaminocarbonyle éventuellement substitué par un atome d'halogène, par un groupe nitro ou par un groupe alkyle en $C_1$-$C_4$, un groupe phényle éventuellement substitué par un atome d'halogène, par un groupe nitro, par un groupe cyano, par un groupe alkyle en $C_1$-$C_4$, par un groupe halogénalkyle en $C_1$-$C_4$ ou par un groupe alcoxy en $C_1$-$C_4$, un groupe benzyle ou un groupe phényléthyle éventuellement substitué par un atome d'halogène, un groupe alcoxy en $C_1$-$C_4$, un groupe alcénoxy en $C_2$-$C_4$, un groupe alcinoxy en $C_2$-$C_4$, un groupe alcoxy (en $C_1$-$C_4$)-carbonylméthoxy, un groupe benzyloxy ou un groupe phénoxy éventuellement substitué par un atome d'halogène, un groupe alkyl (en $C_1$-$C_4$) thio, un groupe alcényl (en $C_2$-$C_4$) thio, un groupe alcinyl (en $C_2$-$C_4$) thio, un groupe alcoxy (en $C_1$-$C_4$) carbonylméthylthio, un groupe benzylthio, un groupe phénylthio, un groupe alkyl (en $C_1$-$C_4$) sulfinyle, ou un groupe alkyl (en $C_1$-$C_4$) sulfonyle éventuellement substitué par un atome d'halogène, un groupe alkyle en $C_1$-$C_6$, un groupe alcényle en $C_2$-$C_6$ ou un groupe alcinyle en $C_2$-$C_6$ éventuellement substitué par un atome d'halogène, un groupe cyanoalkyle en $C_1$-$C_4$, un groupe alcoxy (en $C_1$-$C_4$) alkyle en $C_1$-$C_2$, un groupe phénoxy- ou phénylthiométhyle, un groupe benzyloxy- ou benzylthiométhyle, un groupe alkyl (en $C_1$-$C_4$) thioalkyle en $C_1$-$C_2$, un groupe alkyl (en $C_1$-$C_4$)- ou phénylsulfinylalkyle en $C_1$-$C_2$, un groupe alkyl (en $C_1$-$C_4$)- et phénylsulfonylalkyle en $C_1$-$C_2$, un groupe carboxyalkyle en $C_1$-$C_2$, un groupe alcoxy (en $C_1$-$C_4$) carbonylalkyle en $C_1$-$C_2$, un groupe alkyl (en $C_1$-$C_4$) aminocarbonylalkyle en $C_1$-$C_2$, un groupe dialkyl (en $C_1$-$C_4$) aminocarbonylalkyle en $C_1$-$C_2$, un groupe phénylaminocarbonyl-alkyle ou un groupe cycloalkyle en $C_3$-$C_{12}$,

caractérisé en ce qu'on fait réagir des amides d'acides hydroxyacétiques de formule II :

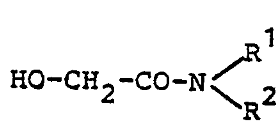

(II)

dans laquelle

$R^1$ et $R^2$ ont les significations indiquées dans la formule I, avec des halogénazoles de formule III :

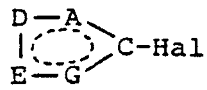

(III)

dans laquelle

A, D, E et G ont les significations indiquées dans la formule I, et

Hal représente un atome de chlore, un atome de brome ou un atome d'iode,

éventuellement en présence d'un accepteur d'acide et en utilisant éventuellement un diluant.

7. Agents herbicides, caractérisés en ce qu'ils contiennent au moins un amide d'acide azolyloxyacétique de formule I suivant la revendication 1 ou 6.

8. Procédé en vue de combattre la croissance inopportune des plantes, caractérisé en ce qu'on fait agir au moins un amide d'acide azolyloxyacétique de formule I suivant la revendication 1 ou 6 sur les plantes non désirées ou leur biotope.

9. Utilisation d'amides d'acides azolyloxyacétiques de formule I suivant la revendication 1 ou 6, pour combattre la croissance non désirée des plantes.

10. Procédé de préparation d'agents herbicides, caractérisé en ce qu'on mélange des amides d'acides azolyloxyacétiques de formule I suivant la revendication 1 ou 6 avec des diluants et/ou des agents tensio-actifs.